# EUROPEAN PATENT APPLICATION

(11) **EP 3 690 046 A2**
(43) Date of publication of application: **05.08.2020**
(21) Application number: 18862743.4
(22) Date of filing: 28.09.2018
(51) Int. Cl.: C12N 15/113, C12N 9/22, C12N 15/10, C12N 15/86, C12N 15/90, A61K 48/00, A61P 7/04

(54) **COMPOSITION FOR TREATMENT OF HEMOPHILIA, COMPRISING CRISPR/CAS SYSTEM HAVING COAGULATION FACTOR VIII GENE INVERSION CORRECTION POTENTIAL**

(30) Priority: 28.09.2017 US 201762564708 P
(71) Applicant: Toolgen Incorporated, Seoul 08501 (KR)
(72) Inventor: KIM, Seokjoong, Seoul 06622 (KR); SONG, Dong Woo, Seoul 08532 (KR); LEE, Kyu Jun, Seoul 08714 (KR); LEE, Jung-Min, Seoul 07340 (KR)
(74) Representative: Mewburn Ellis LLP
(86) International application number: PCT/KR2018/011480
(87) International publication number: WO 2019/066518

(57) **Abstract**

The present invention relates to a CRISPR/Cas system having an inversion correction potential, which uses at least one guide RNA targeting a sequence region where two different homologs present on genomic introns are conjugated to each other in an inversion manner, and a Cas protein, and a CRISPR/Cas system of FVIII gene inversion correction potential that uses at least one guide RNA targeting an int22-1/3 homolog or int22-1/2 homolog sequence region present on intron 22 of coagulation factor VIII (F8) gene and a Cas protein. A CRISPR/Cas system according to the present invention comprises a system which employs a small-size Cas9 and a guide RNA fitted thereto, thereby enabling all CRISPR/Cas instruments to be easily packaged in one AAV, which is impossible in conventional large-size Cas9. In addition, the CRISPR/Cas system can induce normal gene expression thanks to the inversion gene correction potential thereof and is excellent as a technology capable of effectively overcoming the difficult intracellular delivery of large-size gene mutation through gene editing. Particularly, the system can induce normal FVIII expression by restoring the inversion of FVIII gene and thus is useful for the treatment of hemophilia A.

## Description

### [Technical Field]

The present invention relates to a CRISPR/Cas system which has the activity of restoring an inversion by targeting homologous regions of inverted genes, and more particularly to a CRISPR/Cas system capable of restoring inversion mutations occurring at intron 22 of the FVIII gene, and a technology for treating hemophilia A using the same.

### [Background Art]

Hemophilia is a congenital hemorrhagic disease caused by congenital deficiency in blood coagulation factors, and it is known that hemophilia A is caused by factor VIII (FVIII) deficiency, hemophilia B is caused by factor IX (FIX) deficiency (Christmas disease), and hemophilia C is caused by factor XI (FXI) deficiency.

Hemophilia A and B, which account for 95% of hemophilia-related symptoms, are inherited in a recessive X-chromosome-linked manner, in particular, the incidence of hemophilia A is 1 in every 5,000 to 10,000 boys, and 20 to 30% of patients have no family history and have hemophilia due to mutations, and in Korea, hemophilia A is about 6 times more prevalent than hemophilia B.

Hemophilia A is classified depending on the activity of the blood coagulation factor FVIII as mild (5-30% activity), moderate (1-5% activity), and severe (<1% activity), and clinically, bleeding continues after external injury, tooth extraction, and surgical operation, and diagnosis is possible within one year after birth (*J Graw* et al., 2005). Depending on the symptoms, severe type A causes spontaneous bleeding within 2 to 5 months if not treated, while mild type A does not cause spontaneous bleeding unless surgical damage occurs and may go undiagnosed throughout one's entire lifetime.

For the bleeding pattern of hemophilia A, joint bleeding is the most common, accounting for about 60% of cases, and the severe type starts to occur at 12 to 18 months, at which babies generally start to walk, and the moderate type occurs at around 2 to 5 years old. Repeated joint bleeding causes chronic synovial thickening, which eventually damages cartilage, causes stiffening of joints due to osteocystoma formation and bone erosion, and results in limited movement and deformation. Intramuscular bleeding, usually caused by physical injury or intramuscular injection, accounts for about 30% of bleeding, and bleeding in confined spaces causes hematoma, thereby pressing critical organs, leading to sensory and motor disorders and arterial circulation disorders. Unless it is treated immediately, permanent deformation may occur, submucosal bleeding such as bleeding of the tongue and throat can block the airways and be life-threatening, gastrointestinal bleeding usually results from lesions in the upper gastrointestinal tract, and intracranial hemorrhaging is a common cause of death and occurs in about 10% of patients after injury, with death in 30% thereof.

Hemophilia was treated only with plasma and whole blood until the 1960s, and since it was discovered in 1965 that cryoprecipitate contained a large amount of FVIII factor, the concentrate was developed as a therapeutic agent, and in 1987, recombinant FVIII started to be used as a therapeutic agent, but since there is actually no therapeutic agent for fundamental treatment, the treatment of hemophilia is combined with bleeding-related treatment and treatment for complications.

FVIII, which is a hemophilia-A-related blood coagulation factor, is present at the end of the long arm of the X-chromosome (Xq28), is a gene having a full length of 186 kb which consists of 26 exons and 25 introns, and is a huge gene that accounts for 0.12% of the X chromosome. It is known that 50% of severe hemophilia A is caused by inversion mutations of intron 22 (int22), and moderate and mild hemophilia A are caused by point mutations.

Inversion of FVIII occurs as a result of recombination by homologs present on introns 1 and 22, with about 5% of inversion of intron 1 and about 40% of inversion of intron 22, which means that inversion by intron 22 occurs about 8 times more frequently. For intron 22, it is known that a nucleic acid fragment of approximately 500-600 kb in length could be invertedly mutated derived from homologous recombination of the int 22h-1 homolog present on the intron and int22h-2 (type 2) or int22h-3 (type 1) located away towards the telomeric side by approximately 500 kb (Naylor J et *al.,* 1995).

Recently, a method of reversing (restoring) an inversion mutation state of int1 in FVIII using zinc finger nuclease (ZFN) or transcriptional activator-like effector nuclease (TALEN) (Park et *al.,* 2014), and a method of restoring int22-1/3 inversion using CRISPR-Cas9 (Park et al., Cell Stem Cell, 2015) have been developed, and gene-editing techniques have begun to be used to restore the FVIII gene.

The CRISPR/Cas system is originally an adaptive immune system from bacteria or archaea for the introduction of foreign genes, such as viruses and phages (Pendades et *al.,* 2014). The CRISPR/Cas system is divided into Type I, II and III according to the composition of protein complexes including the Cas protein constituting the system, and is subdivided according to the composition and number of Cas genes (Makarova, A et *al.,* 2011). Thereamong, the Type II CRISPR/Cas system derived from *Streptococcus pyogenes,* which is being studied most actively because the system is suitable for use as a gene-editing technique due to the small size of an operon thereof, consists of Cas9, forms a complex with two types of RNA, namely crRNA and tracrRNA, and recognizes the PAM sequence present on a target gene to distinguish the target, and has activity of generating a double-strand break therefor (Jinek et *al.,* 2012).

Specifically, a gene-editing mechanism using the CRISPR/Cas system proceeds by a complex of Cas9 and (a) crRNA and tracrRNA which contain a guide sequence that recognizes a target gene of less than 30 bp or (b) a single-stranded guide RNA fused with the double strands of RNAs. When Cas9 in the complex recognizes a protospacer adjacent motif (PAM) present on the target gene and is located on double-stranded DNA, a guide sequence of crRNA binds to a sequence complementary to the target sequence present on the 5' (upstream) side of the PAM, and a double-strand break (DSB) is produced by Cas9. The cleaved DNA undergoes genetic mutations or gene correction by non-homologous end joining (NHEJ) or homologous recombination (homolog-directed repair), which is a DNA repair system present in cells. Non-homologous end joining is efficient, but randomly rearranged genes result in insertion/deletion mutations, and homologous recombination mechanisms that can occur when DNA donors containing sequences homologous to sequences near the cleaved DNA are present are inefficient, but enable accurate gene correction.

The protospacer adjacent motif (PAM) has different sequences depending on the Cas protein that recognizes the same and, for example, it is known that *S. pyogenes-derived* Cas9 is 5'-NGG-3' (N being one of A, T, G, and C), *S. thermophilus*-derived Cas9 is 5'-NNAGAAW-3', *C jejuni*-derived Cas9 is 5'-NNNNRYAC-3', and since the sequences are arranged at regular intervals on the human genome, the CRISPR-Cas system can be used in gene editing.

To develop a therapeutic agent using the gene editing technique, it is important to minimize target delivery capacity and an off-target effect. In the former case, the development of technology for improving the efficiency of *in-vivo* delivery continues, and *ex-vivo* techniques are also used. In the latter case, a verification system is constructed to enable the selection of guide RNAs that recognize a target having minimized or no off-target effect using various verification methods such as Digenome-seq, Guide-seq, and Bless in a process of designing guide RNAs.

In particular, the *ex-vivo* type gene correction method is efficient in terms of targeted delivery ability, but since these cell therapy methods are difficult to use to obtain a fundamental gene treatment effect, the number of diseases treatable thereby is limited, and thus *in-vivo* gene correction is essential in order to treat various diseases. Representative techniques that can be utilized as an *in-vivo* carrier include a method using a viral vector such as an adeno-associated virus (AAV) and a method using a non-viral vector constructed by packaging Cas9 mRNA and sgRNA into lipid nanoparticles. In particular, AAV, which is a viral vector found to be safe for use as a carrier through various clinical trials and the development of Glybera, is very useful for gene delivery, but has a small amount of genetic information that can be packaged in a single viral vector. In particular, for the delivery of the CRISPR-Cas system, since it is difficult for the Cas protein and guide RNA, which have large molecular weights, to be simultaneously packaged in a single AAV, Cas9, having a low molecular weight, is selected as an alternative for technological development.

To develop a CRISPR-Cas system having gene inversion correction potential, as a result of having induced reversion by applying, to an inverted state of FVIII, which is a gene causative of hemophilia A, a CRISPR-Cas system using a guide RNA targeting a sequence region of homolog 1/3 or 1/2 of intron 22 of the gene and having confirmed gene restoration efficiency and enzymatic activity in the blood, the inventors of the present invention confirmed that FVIII having normal activity was expressed by the gene restoration of FVIII, and thus completed the present invention.

### [Disclosure]

### [Technical Problem]

Therefore, the present invention has been made in view of the above problems, and it is an object of the present invention to provide a CRISPR/Cas system for editing an inversion, which includes at least one guide RNA targeting a sequence region where two different homologs present in an intron on the genome are inverted to be conjugated, and a Cas protein.

It is another object of the present invention to provide a CRISPR/Cas system for editing an inversion, which includes at least one guide RNA targeting a sequence region of an int22-1/2 homolog or int22-1/3 homolog resulting from conjugation by inversion between homolog 1 (int22-1) and homolog 2 (int22-2) or between homolog 1 (int22-1) and homolog 3 (int22-3) of intron 22 in the blood coagulation factor VIII (F8) gene.

### [Technical Solution]

In accordance with an aspect of the present invention, the above and other objects can be accomplished by the provision of a CRISPR/Cas system for editing an inversion by using at least one guide RNA targeting a sequence region where two different homologs present in an intron on the genome are inverted to be conjugated, and a Cas protein.

In accordance with another aspect of the present invention, there is provided a CRISPR/Cas system for editing an inversion of the blood coagulation factor VIII (FVIII) gene by using at least one guide RNA targeting a sequence region of an int-1/3 homolog or int22-1/2 homolog present in intron 22 in the blood coagulation factor VIII gene, and a Cas protein.

In accordance with a further aspect of the present invention, there is provided a CRISPR/Cas system for editing an inversion of the blood coagulation factor VIII (FVIII) gene by using a guide RNA that specifically targets one or more nucleic acid sequences selected from sequences represented by SEQ ID NOS: 1 to 42, and a Cas protein.

In accordance with a further aspect of the present invention, there is provided a composition for editing an inversion of the blood coagulation factor VIII (F8) gene, which includes: (i) a Cas protein or a nucleotide encoding the Cas protein; and (ii) at least one guide RNA that specifically targets a sequence region of an int22-1/3 homolog or int22-1/2 homolog resulting from conjugation by inversion between homologs 1 (int22-1) and 2 (int22-2) or between homologs 1 (int22-1) and 3 (int22-3) of intron 22 in the blood coagulation factor VIII (F8) gene.

In accordance with a further aspect of the present invention, there is provided a composition for the prevention or treatment of hemophilia, which includes: (i) a Cas protein or a nucleotide encoding the Cas protein; and (ii) at least one guide RNA that specifically targets a sequence region of an int22-1/3 homolog or int22-1/2 homolog resulting from conjugation by inversion between homologs 1 (int22-1) and 2 (int22-2) or between homologs 1 (int22-1) and 3 (int22-3) of intron 22 in the blood coagulation factor VIII (F8) gene.

In accordance with a further aspect of the present invention, there is provided a composition for inducing an inversion of the blood coagulation factor VIII (F8) gene, which includes: (i) a Cas protein or a nucleotide encoding the Cas protein; and (ii) at least one guide RNA that specifically targets a sequence region of an int22-1/3 homolog or int22-1/2 homolog resulting from conjugation by inversion between homologs 1 (int22-1) and 2 (int22-2) or between homologs 1 (int22-1) and 3 (int22-3) of intron 22 in the blood coagulation factor VIII (F8) gene.

In accordance with a further aspect of the present invention, there is provided a guide RNA that specifically targets one or more sequences selected from sequences represented by SEQ ID NOS: 1 to 42.

### [Description of Drawings]

The above and other objects, features and other advantages of the present invention will be more clearly understood from the following detailed description taken in conjunction with the accompanying drawings, in which:

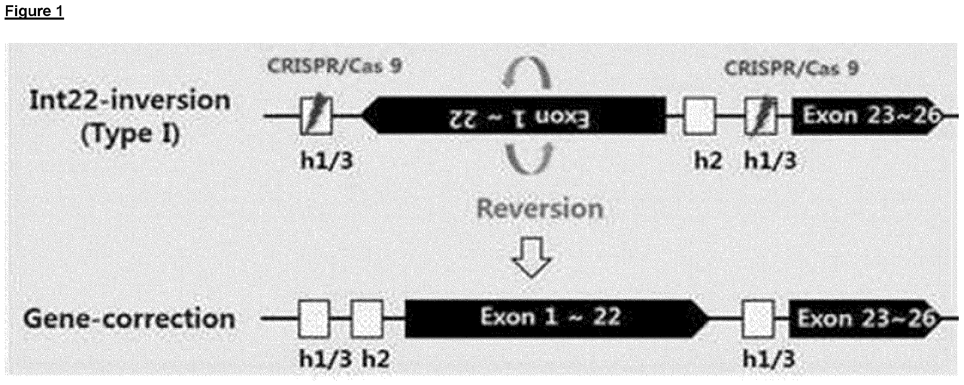

FIG. 1 is a view for an inversion restoration strategy of intron 22 (int22) of the blood coagulation factor VIII (FVIII) using a CRISPR/Cas9 system;

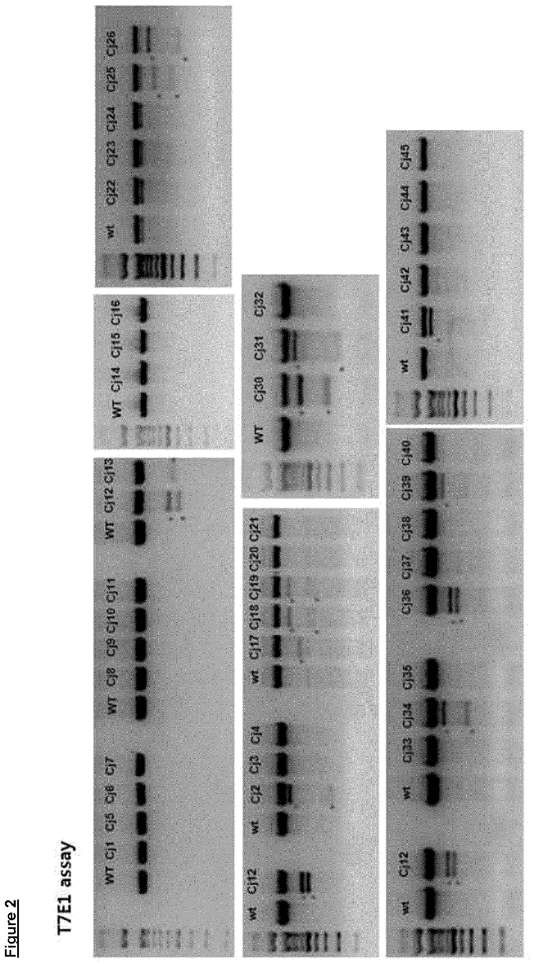

FIG. 2 illustrates the results showing the cleavage capacity of a CRISPR/Cas system by a guide RNA targeting an int22-1/3 or int22-1/2 homolog sequence of hFVIII;

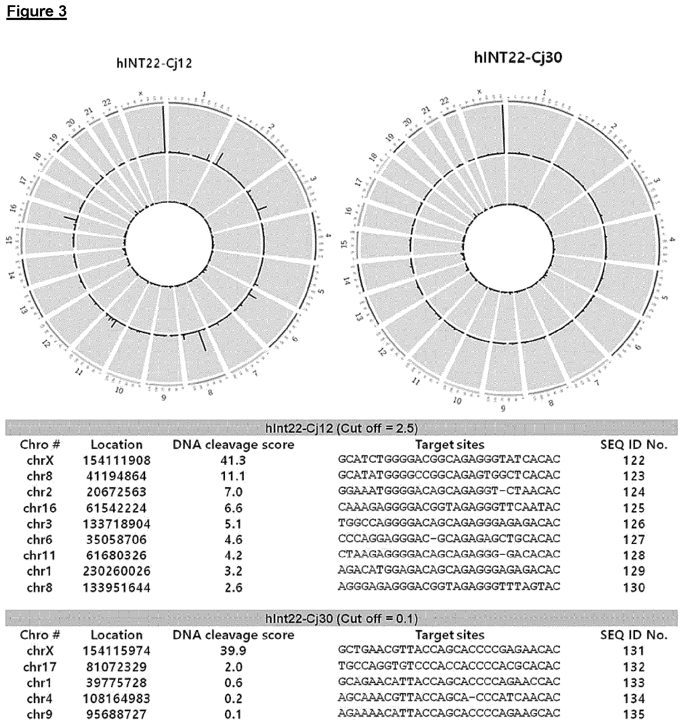

FIG. 3 illustrates targeted deep-sequencing results confirming the activity of the selected guide RNAs on off-targets;

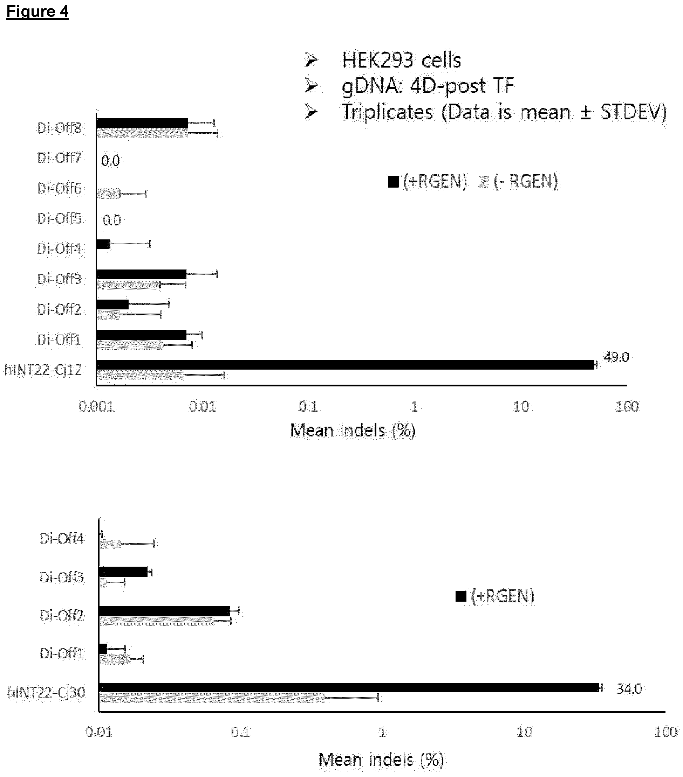

FIG. 4 is a set of graphs showing the results of comparing off-target effects of the selected guide RNAs;
FIG. 5 illustrates next-generation sequencing (NGS) results confirming the activity of selected guide RNAs on off-targets;

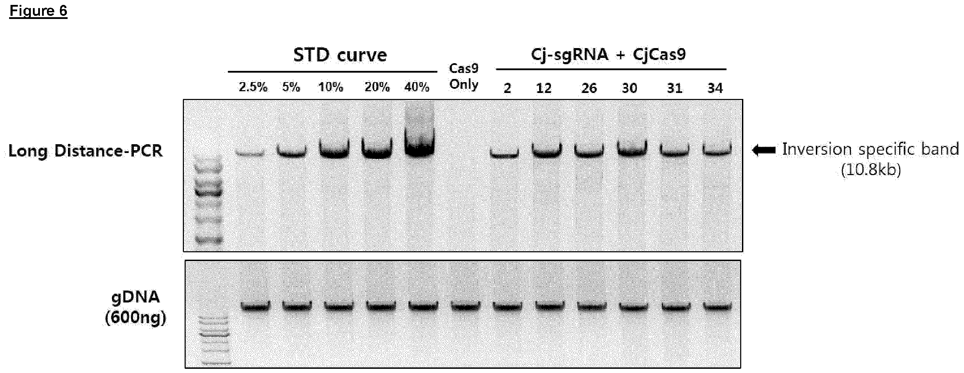

FIG. 6 illustrates the results of verifying cleavage efficiency by a guide RNA targeting the int22-1/3 or int22-1/2 homolog sequence of hFVIII; and

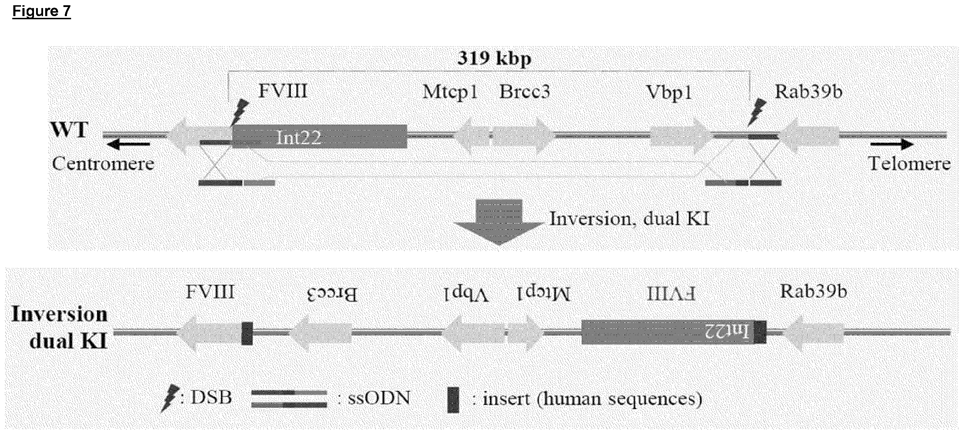

FIG. 7 illustrates the results of confirming a gene insertion plan for producing an animal model including an intron 22 (int22) inversion gene of human FVIII, and an inserted state thereof.

### [Detailed Description and Exemplary Embodiments]

Unless defined otherwise, all technical and scientific terms as used herein have the same meanings as commonly understood by one of ordinary skill in the art to which the present invention pertains. Generally, the nomenclature used herein is well known in the art and is commonly used.

The present invention relates to a composition for editing an inversion of the blood coagulation factor VIII (F8) gene, which includes: (i) a Cas protein or a nucleotide encoding the Cas protein; and (ii) at least one guide RNA that specifically targets a sequence region of an int22-1/3 homolog or int22-1/2 homolog resulting from conjugation by inversion between homologs 1 (int22-1) and 2 (int22-2) or between homologs 1 (int22-1) and 3 (int22-3) of intron 22 in the blood coagulation factor VIII (F8) gene.

In relation to the development of a therapeutic agent for hemophilia A, to restore an inverted state of the FVIII gene using the CRISPR/Cas system, it is required to design a guide RNA targeting the sequence of the int22-1/3 homolog or the int22-1/2 homolog, which is a conjugated region between homolog 1 and homolog 2 or 3 of int22, which is an inversion position.

Generally, in guide RNAs, a guide sequence having a length of 30 bp or less present on the 5' end thereof binds to a target site, and the 3' end thereof is involved in forming a complex with the Cas protein, which is a nuclease, and since the Cas protein-binding site on the 3' end has a unique sequence due to the biological characteristics thereof, designing a guide RNA means a process of selecting a guide sequence that binds to the target site.

The term "target of the guide RNA" as used herein refers to a sequence on the genome that is cleaved by the CRISPR/Cas system or undergoes modification such as deletion, insertion, or substitution in a restoration process after cleavage, and to which a guide sequence binds. For example, in the present invention, the target is a gene region capable of being hybridized with the guide RNA in the int22-1/2 homolog or int22-1/3 homolog sequence region, and is a consecutive nucleotide sequence having a length of 17 bp to 23 bp or 18 bp to 22 bp located adjacent to the 5' end and/or the 3' end of a protospacer-adjacent motif (PAM) present on DNA including the int22-1/2 homolog or int22-1/3 homolog sequence region. For example, an upstream sequence on the 5' end is set as a guide sequence, and the guide sequence may be designed to be positioned on the 5' end of the guide RNA. Actually, since the guide RNA hybridizes to a strand complementary to a DNA strand where the PAM is present, the sequence of the guide RNA has the same characteristics as those of the upstream sequence of the 5' end of the PAM.

The sequence of the guide RNA that can be hybridized with the target region of the target gene is a nucleotide sequence with at least 90% homology, at least 95% homology, at least 96% homology, at least 97% homology, at least 98% homology, at least 99% homology, or 100% homology to a nucleotide sequence of a strand complementary to a DNA strand at which the target sequence is positioned (i.e., a DNA strand at which the PAM sequence is positioned), and is capable of binding to the nucleotide sequence of the complementary strand.

The target sequence is represented by a nucleic acid sequence of a strand where the PAM sequence is located, among the two DNA strands of the int22-1/2 homolog or int22-1/3 homolog sequence region, which is a target gene. In this regard, since the DNA strand to which the guide RNA binds is actually a strand complementary to the strand where the PAM sequence is located, a sequence included in the guide RNA has the same nucleic acid sequence as the target sequence located in the target gene, except that T is changed to U due to the characteristics of RNA. Thus, in the present specification, the guide RNA sequence and the target sequence are represented by the same nucleic acid sequence, except that T is changed to U or vice versa.

The target sequence has very high genetic correction efficiency (e.g., indel frequency (%)) at on-target sites, and at sites except for the on-target sites, since the number of mismatching nucleotides is 3 or less, 2 or less, 1, or 0, there are almost no off-target sites, and thus there is little or no chance that genetic correction will occur in regions except for the on-target sites, such that the target sequence has excellent safety.

The guide RNA may be capable of being hybridized with a target including, a proto-spacer-adjacent motif (PAM) sequence 5'-NNNNRYAC-3' and for example, a sequence comprising 1 bp or 2 bp mismatch not present on a human genome.

In one embodiment, the guide RNA includes a sequence that specifically targets a target including a sequence selected from SEQ ID NOS: 1 to 42 and is hybridizable with the target, and the guide RNA may include one or more sequences selected from sequences represented by SEQ ID NOS: 1 to 42 (provided that T in the sequence is changed to U).

Since the specificity of the CRISPR/Cas system is determined by the guide sequence of the guide RNA, an activity verification process must be performed before application to a gene-editing process: guide sequences are selected through an available program such as ATUM Scoring Algorithms or GenScript based on the protospacer-adjacent motif (PAM) present in the target gene, guide RNAs including the selected guide sequences are further subjected to mismatch-sensitive T7 endonuclease I (T7E1) analysis and targeted deep-sequencing to verify on-target and off-target effects, and finally, guide RNAs with high target specificity and no off-target effect are determined.

In the present invention, for 10 kb of the int22-1/2 homolog or int22-1/3 homolog sequence, for example, about 42 guide sequences where 1b-mismatched off-target or 2b-mismatched off-target is not present on the human genome were designed by considering the PAM sequence 5'-NNNNRYAC-3' (each N independently being A, T, C, or G, R being A or G, and Y being C or T) for the Cas protein derived from *Campylobacter jejuni,* guide RNAs including the same were produced, and guide RNAs with excellent gene cleavage activity were selected through T7E1 assay and verification of each of the produced guide RNAs.

As a result of measuring the frequency of induction of an inversion of the FVIII gene in HEK293 cells, which are normal cells, 6 types with the highest cleavage activity among the selected guide RNAs of the present invention were found to have an efficiency of 5% to 10%. This result contrasted notably with a frequency of inversion induction of 1.5% to 2.2% in a Hela cell line, as disclosed in the related art (Park et al., Cell Stem Cell, 2015) which discloses targeting the external sequence of the int22-1/3 homolog with two guide RNAs, from which it was confirmed that the guide RNA of the present invention has considerably high activity. In particular, it was demonstrated that the CRISPR/Cas system of the present invention is capable of reversing an inversion of the FVIII gene using only a guide RNA and the Cas protein, unlike the related art, which uses two types of guide RNAs.

Since the sequence targeted by the guide RNA in the normal cells is the same sequence as that of a target for restoring the inverted FVIII gene, it will be obvious to those of ordinary skill in the art to predict gene restoration capability based on the frequency of inversion induction in normal cells rather than in a disease model.

An embodiment of the present invention relates to a CRISPR/Cas system having inversion correction capability, which uses at least one guide RNA targeting a sequence region where two different homologs present in an intron on the genome are inverted and conjugated, and the Cas protein.

As used herein, the term "intron" is a nucleic acid site present on the genome of eukaryotic cells, which is present in pre-mRNA during transcription, but is removed during matured mRNA production by splicing, and thus is a DNA sequence on the genome that is not involved in protein production.

As used herein, the term "homolog" is a section in which the same sequence is repeated on an intron, and may be interchangeably used with the term "homologous copy" or "homolog repeat." Since homologs present at different positions on the genome have the same sequence, the homologs may cause homologous recombination during a genetic recombination process, and in the FVIII gene, representative examples of the homologs include an int22-1 homolog, an int22-2 homolog, and an int22-3 homolog. For reference, the sizes of the respective homologs of FVIII are 10 kb, 12 kb, and 11 kb, respectively.

As used herein, the term "inversion" is a structural abnormality of a chromosome caused by rearrangement of sequences generated by homologous recombination between different homologs on the chromosome. Most mutations caused by an inversion exhibit a normal phenotype, but when occurring in the meiosis stage of germ cells, such mutations may cause the formation of a recombinant chromosome through deletion or duplication of chromosomes, thus exhibiting an abnormal phenotype. Most commonly, there is hemophilia A caused by an inversion at 28q of the X chromosome, and there are also known diseases caused by inversions, such as Wolf-Hirschhorn's syndrome, in which 4p is deleted due to a structural abnormality in chromosome 4, and Hunter's disease, caused by an inversion of the IDS gene in the Xq chromosome.

As used herein, the term "conjugation" refers to a state in which a nucleic acid sequence artificially cleaved by a genetic recombination process or endonuclease is relinked, and can be used interchangeably with "ligation".

As used herein, the term "cleavage" refers to breakage of the covalent backbone of a DNA molecule, and may be initiated by various methods, including enzymatic or chemical hydrolysis of a phosphate bond. Both single-strand cleavage and double-strand cleavage are possible, among which double-strand cleavage may occur as a result of cleaving two separate single strands. DNA cleavage may result in the production of blunt ends or staggered ends, and "cleavage" in the present invention refers to a blunt end.

The guide RNA for reversing an inversion according to the present invention targets the sequence region resulting from conjugation by inversion. The term "sequence region resulting from conjugation" refers to a range corresponding to the length of a recombinant homolog present on the genome, and particularly, may be a range including a sequence having a maximum size of 12 kb including the 5' and 3' directions with respect to the conjugated position.

Generally, the CRISPR/Cas system consists of the Cas protein, trans-activating RNA (tracrRNA), and crRNA, and the crRNA includes a spacer that binds to a target site and an RNA sequence capable of being hybridized with tracrRNA, and thus is first hybridized with tracrRNA before forming a complex with the Cas protein, thereby forming an RNA-RNA conjugate. In addition, crRNA and tracrRNA may be artificially fused and induced to form a complex with the Cas protein in the single-stranded state.

As used herein, the term "guide RNA" generally includes tracrRNA and crRNA, and specifically, refers to double-stranded RNA consisting of RNA including a spacer or guide sequence that binds to a target site (sequence) and RNA that binds to the Cas protein, or single-stranded RNA obtained by fusing said two RNAs. Thus, the term "guide RNA" may be used interchangeably with the terms "single-stranded guide RNA," "chimeric RNA," "chimeric guide RNA," and "synthetic guide RNA." In addition, any RNA that binds to the Cas protein (or to an endonuclease) to bind to the target site, or binds to the same to form a complex having a characteristic of cleaving the target site may be interpreted as being encompassed by the term "guide RNA."

A "fused" molecule is a molecule in which two or more subunit molecules are preferably covalently linked. The subunit molecules may be molecules of the same chemical type or molecules of different chemical types. The fusion of crRNA and tracrRNA in the guide RNA refers to a state in which the backbone is connected by phosphorylation bonds, not hydrogen bonds, and consists of a single strand.

As used herein, the term "guide sequence" refers to an RNA sequence having a length within 30 bp at the 5' end of the guide RNA and having characteristics complementary to a target site, and may be used interchangeably with the term "spacer" or "spacer sequence." Generally, all sequences that are not involved in binding to the Cas protein in the Cas protein/guide RNA complex and complementarily bind to the target site may be interpreted as being encompassed by the above term.

The guide sequence may be easily selected by one of ordinary skill in the art using CRISPR RGEN Tools (http://www.rgenome.net; Park et al, Bioinformatics, 31:4014-4016, 2015); sgRNA Designer (Doench et al., Nature Biotechnology 34(2) : 184-191, 2016); E-CRISP (http://www.e-crisp.org/E-CRISP/Heigwar et al., Nature Methods 11(2):122-123, 2014); Benchling (https://benchling.com); sgRNA scorer 2.0 (https://crispr.med.harvard.edu/sgRNAScorerV2; Chari et al., ACS Synthetic Biology, 2017. doi: 10.1021/acssynbio.6b00343), and CRISPy-web (Blin et al., Synthetic and Systems Biotechnology, 1(2): 118-121, 2016).

In the present invention, the guide RNA may consist of (a) crRNA including a guide sequence and tracrRNA, (b) a fusion body of crRNA including a guide sequence and tracrRNA (chimeric guide RNA), or (c) crRNA including a guide sequence, but the present invention is not limited thereto.

For the guide RNA of the present invention, modifications may be applied to nucleic acid backbones or bases: in backbone modifications, phosphorothioates, chiral phosphorothioates, phosphorodithioates, phosphotriesters, aminoalkylphosphotriesters, 3'-alkylene phosphonates, 5'-alkylene phosphonates, chiral phosphonates, phosphinates, phosphoramidates including 3'-amino phosphoramidate aminoalkylphosphoramidates, phosphorodiamidates, thionophosphoramidates, thionoalkylphosphonates, thionoalkylphosphotriesters, selenophosphates, and boranophosphate may be used, and in sugar molecule residue modifications, 2'-methoxyethoxy, 2'-MOE, 2'-dimethylaminooxyethoxy, 2'-dimethylaminoethoxyethoxy, methoxy, aminopropoxy, fluoro, ally, and -O-allyl may be used, but the present invention is not limited thereto. In addition, in base modifications, instead of generally using purine and pyrimidine, 5-methylcytosine(5meC), 5-hydroxymethyl cytosine, xanthine, hypoxanthine, 2-aminoadenine, 7-deaza-adenine, 7-deazaguanosine, 2-aminopyridine, 2-pyridone, 2-thiouracil, 2-thiothymine 2-thiocytosine, 5-halouracil, 5-propynyl uracil, 6-azo uracil, pseudouracil, 7-methylguanine, 7-methyladenine, 2-F-adenine, 2-aminoadenine, 8-azaguanine, 8-azaadenine, 7-deazaguanine, 7-deazaadenine, 3-deazaguanine, 3-deazaadenine, phenoxazine cytidine, phenothiazine cytidine, carbazole cytidine, and pyridoindole cytidine may be used, but the present invention is not limited thereto.

The Cas protein of the present invention may be derived from a microorganism including an ortholog of the Cas protein selected from the genus *Corynebacter,* the genus *Sutterella,* the genus *Legionella,* the genus *Treponema,* the genus *Filifactor,* the genus *Eubacterium,* the genus *Streptococcus,* the *genus Lactobacillus,* the genus *Mycoplasma,* the genus *Bacteroides,* the genus *Flaviivola,* the genus *Flavobacterium,* the genus *Azospirillum,* the genus *Gluconacetobacter,* the genus *Neisseria,* the genus *Roseburia,* the genus *Parvibaculum,* the genus *Staphylococcus,* the genus *Nitratifractor,* the genus *Corynebacterium,* and the genus *Campylobacter,* and may be at least one selected from simply isolated Cas proteins and recombined Cas proteins, particularly a Cas protein derived from the genus *Campylobacter,* but the present invention is not limited thereto.

The Cas protein of the present invention binds to the guide RNA and forms a complex, recognizes the PAM sequence present on the sequence of the target gene to guide the CRISPR/Cas system to the target gene, recognizes the target gene through complementary binding between the guide sequence of the guide RNA and the target site, and finally exhibits nucleic acid cleavage activity having a characteristic of a double-strand break due to active sites of Cas9, e.g., the HNH domain and the RuvC domain thereof.

Gene and protein information of the Cas protein can be obtained from GenBank of the National Center for Biotechnology Information (NCBI).

In the present invention, the Cas protein may be a nuclease selected from Cas3, Cas9, Cpf1, Cas6, and C2c2, particularly the Cas protein of CRISPR/Cas type II, and more particularly a *Campylobacter* jejuni-derived Cas protein.

In the present invention, the Cas protein may be a nuclease simply isolated from a microorganism, but may be modified to (a) a variant in which some amino acid sequences are substituted, (b) a fusion protein with an affinity tag, recombinase, transposon, or the like, (c) a variant to which nuclear localization sequences (NLS) are added, or the like.

The CRISPR/Cas system according to the present invention enables normal genes to be expressed by editing inverted genes, and thus may induce a semi-permanent treatment effect on a disease. The proportion of normal gene expression may range from 1% to 100%, particularly 1% to 80%, and more particularly 30% to 50%, but the present invention is not limited thereto.

At least one guide RNA and the Cas protein, which are detailed components of the CRISPR/Cas system of the present invention, may be intracellularly delivered via (a) a vector including nucleic acid sequences expressing at least one guide RNA and the Cas protein, (b) a ribonucleoprotein (RNP) or RNA-guided engineered nuclease (RGEN) consisting of at least one guide RNA and the Cas protein, or (c) a configuration of at least one guide RNA and mRNA by which the Cas protein is encoded, but the present invention is not limited thereto. The vector including nucleic acid sequences expressing at least one guide RNA and the Cas protein may simultaneously include at least one guide RNA and the Cas protein, or may consist of two or more vectors respectively expressing the guide RNA and the Cas protein.

In the CRISPR/Cas system of the present invention, the vector may be one or more vectors, and may be a plasmid vector or a viral vector, and the viral vector may particularly be an adenovirus vector, an adeno-associated virus vector, a lentivirus vector, or a retrovirus vector, but the present invention is not limited thereto.

The vectors of the present invention may be delivered to a cell by microvehicles, liposomes, exosomes, nanoparticles, a gene gun, or the like, but the present invention is not limited thereto, and it will be obvious to those of ordinary skill in the art to select a delivery method suitable for the structural characteristics of vectors and the characteristics of cells to which the vectors are to be delivered.

To verify that the CRISPR/Cas system can restore inverted genes and enable normal gene expression, thus inducing therapeutic effects, the inventors of the present invention determined the restoration capacity by the CRISPR/Cas system via targeting a sequence region of the int22-1/3 homolog of the FVIII gene causative of hemophilia A, which is a representative disease caused by genetic inversions, as follows.

The inventors of the present invention first selected, from the 10 kb sequence of an int22-1/2 homolog or an int22-1/3 homolog of FVIII, which has the PAM sequence 5'-NNNNRYAC-3', about 42 guide RNA sequences where 1b-mismatched off-target or 2b-mismatched off-target is not present on the human genome (see Tables 1 and 2).

**[Table 1]**

| | **guide RNA w/o PAM** | **SEO ID NO** | **Direction** | **mismatch(0)** | **mismatch(1)** | **mismatch(2)** | **Activity (T7E1)** | **Indels in NGS *(* %)** |
|---|---|---|---|---|---|---|---|---|
| hFS-int22-Cj1 | TCTCGTTGGTGT TGTCTCAATG | SEQ ID NO:1 | + | 3 | 0 | 0 | - | |
| hF8-int22-Cj2 | GGTATACTGTGT TAAGCATTGA | SEQ ID NO.:2 | - | 3 | 0 | 0 | ++ | 13.3 |
| hF8-int22-Cj3 | TTAGGTAGTAA AATGGCACACA | SEQ ID NO.:3 | | 3 | 0 | 0 | - | |
| hF8-int22-Cj4 | TCAGGTTTTAG AATACCCTGTA | SEQ ID NO.:4 | - | 3 | 0 | 0 | - | |
| hF8-int22-Cj5 | CTTCTCATCATG CTACAGATTC | SEQ ID NO.:5 | + | 3 | 0 | 0 | - | |
| hF8-int22-Cj6 | TCTCATCATGCT ACAGATTCTT | SEQ ID NO.:6 | + | 3 | 0 | 0 | - | |
| hF8-int22-Cj7 | TCATCATGCTAC AGATTCTTTA | SEQ ID NO.:7 | + | 3 | 0 | 0 | - | |
| hF8-int22-Cj8 | CAGGGTTGGCA TATTAGTCCGT | SEQ ID NO.:8 | - | 3 | 0 | 0 | - | |
| hF8.int22.Cj9 | AATCTGCAACA GTCGGTTGTAT | SEQ ID NO.:9 | + | 3 | 0 | 0 | - | |
| hF8-int22-Cj10 | TGAAATTTTCA GAGTATACATC | SEQ ID NO.:10 | | 3 | 0 | 0 | - | |
| hF8-int22-Cj11 | AAGGCATTTTG TGTTAACGGAT | SEQ ID NO.:11 | + | 3 | 0 | 0 | - | |
| hF8-int22-Cj12 | GGCATCTGGGG ACGGCAGAGGG | SEQ ID NO.:12 | + | 3 | 0 | 0 | +++ | 42 |
| hF8-int22-Cj13 | ATAGGAAGAGA GGCCTGAAACG | SEQ ID NO.:13 | - | 3 | 0 | 0 | ++ | 24.7 |
| hF8-int22-Cj14 | CACTACCAGCCC CCACACCCTC | SEQ ID NO.:14 | - | 3 | 0 | 0 | - | |
| hF8-int22-Cj15 | ACCACTACCAG CCCCCACACCC | SEQ ID NO.:15 | - | 3 | 0 | 0 | - | |
| hF8-int22-Cj16 | CAGTCACTTGCC GCACGCCTCC | SEQ ID NO.:16 | | 3 | 0 | 0 | - | |
| hF8-int22-Cj17 | GAACTTTCCCTT TCTACTGGAT | SEQ ID NO.:17 | - | 3 | 0 | 0 | + | |
| hF8-int22-Cj18 | TGTGGTGACGG CCCCTCACAAG | SEQ ID NO.:18 | - | 3 | 0 | 0 | + | |
| hF8-int22-Cj19 | CATGTGGTGAC GGCCCCTCACA | SEQ ID NO.:19 | - | 3 | 0 | 0 | + | |
| hF8-int22-Cj20 | AGGGGCTGGGC CCTGGTCGCTA | SEQ ID NO.:20 | + | 3 | 0 | 0 | - | |
| hF8-int22-Cj21 | GCCTAGCTGGG CCAAGCCGAGA | SEQ ID NO.:21 | + | 3 | 0 | 0 | - | |
| hF8-int22-Cj22 | CGACAGGCTAC TGAGCACCCTT | SEQ ID NO.:22 | + | 3 | 0 | 0 | - | |

**[Table 2]**

| | **guide RNA w/o PAM** | **SEQ ID NO** | **Direction** | **mismatch( 0)** | **mismatch( 1)** | **mismatch( 2)** | **Activity (T7 E1)** | **Indels in N GS (%)** |
|---|---|---|---|---|---|---|---|---|
| hF8-int22-Cj23 | TGGCCCCT GGCGAGG ACTAGCT | SEQ ID NO. :23 | + | 3 | 0 | 0 | - | |
| hF8-int22-Cj24 | CCCCCGGC CTGAACCC CCGGCC | SEQ ID NO. :24 | + | 3 | 0 | 0 | - | |
| hF8-int22-Cj25 | GGGGAGT GGCCCTGG GTGGGAA | SEQ ID NO. :25 | + | 3 | 0 | 0 | + | |
| hF8-int22-Cj26 | GTCTTGAG GCCCACCC GCCCCA | SEQ ID NO. :26 | NO. | 3 | 0 | 0 | +++ | 31.9 |
| hF8-int22-Cj30 | GGCTGAAC GTTACCAG CACCCC | SEQ ID NO.:27 :27 | + | 3 | 0 | 0 | +++ | 35.2 |
| hF8-int22-Cj31 | TTTATAGA CGACGGAC ACCAA | SEQ ID NO. :28 | - | 3 | 0 | 0 | ++ | 22.6 |
| hF8-int22-Cj32 | AGAGAGTC ACATTTTA TAGACG | SEQ ID NO. :29 | | 3 | 0 | 0 | - | |
| hF8-int22-Cj33 | CTGCCTTG CAGTCACG TGATCG | SEQ ID NO. :30 | - | 3 | 0 | 0 | - | |
| hF8-int22-Cj34 | ACGGAACG CCATCCTG CTTTGG | SEQ ID NO. :31 | NO. | 3 | 0 | 0 | ++ | 23 |
| hF8-int22-Cj35 | AAAGCAG GATGGCGT TCCGTTT | SEQ ID NO. :32 | + | 3 | 0 | 0 | - | |
| hF8-int22-Cj36 | AAATGCAT CCTTTGGG CCGAAG | SEQ ID NO. :33 | + | 3 | 0 | 0 | ++ | 21.4 |
| hF8-int22-Cj37 | CTGATCTT CGGGGCTG AGATGT | SEQ ID NO. :34 | + | 3 | 0 | 0 | - | |
| hF8-int22-Cj38 | CCCCTGCA AAAAGAC GCAAAAC | SEQ ID NO. :35 | - | 3 | 0 | 0 | - | |
| hF8-int22-Cj39 | CACTCAAC TCACAGTG ACAACC | SEQ ID NO. :36 | - | 3 | 0 | 0 | + | |
| hF8-int22-Cj40 | CCTCTCCTG TGCTTCCC ATTAC | SEQ ID NO. :37 | - | 3 | 0 | 0 | - | |
| hF8-int22-Cj41 | TGGGTATC TGAGCATA ATTCTA | SEQ ID NO. :38 | + | 3 | 0 | 0 | ++ | 20.2 |
| hF8-int22-Cj42 | ATGTATCA CATCAGTA TCAAAA | SEQ ID NO. :39 | + | 3 | 0 | 0 | - | |
| hF8-int22-Cj43 | TTTAACTT GAAAACA ATTCCAA | SEQ ID NO. :40 | + | 3 | 0 | 0 | - | |
| hF8-int22-Cj44 | TTCGCTGC CACCATTT CCGACG | SEQ ID NO. :41 | - | 3 | 0 | 0 | - | |
| hF8-int22-Cj45 | CGGACTGT GAGTTAAG AATAGT | SEQ ID NO. :42 | - | 3 | 0 | 0 | - | |

Guide RNAs including the target sequences listed in Tables 1 and 2 were produced, and a CRISPR/Cas system including the same was used to verify the cleavage efficiency thereof on the int22-1/2 homolog or the int22-1/3 homolog of FVIII through a T7E1 assay, and as a result, guide RNAs including 13 guide sequences were selected. Thereafter, the off-target sequence of each guide RNA was found to a level of 3b-mismatch, and after confirming that no off-target was present, whether capability to restore inverted genes was realized was confirmed *in vitro.*

Subsequently, to confirm whether *in-vivo* treatment can be induced, an animal model in which an inversion occurred about 300 kb away from FVIII-int22 was produced, and the selected guide RNAs and the Cas protein were packaged in an AAV, which was then injected into the animal model, and as a result of performing genetic analysis, it was confirmed that the inverted FVIII was reversed. In addition, as a result of evaluating the activity of FVIII in blood vessels, it was verified that the activity was increased to a significant level.

Therefore, other embodiments of the present invention relate to a CRISPR/Cas system for editing an inversion of the blood coagulation factor VIII (FVIII) gene, which uses at least one guide RNA targeting a sequence region of an int22-1/3 homolog or int22-1/2 homolog of intron 22 in the blood coagulation factor VIII (FVIII) gene, and a Cas protein, and particularly to a composition for editing an inversion of the blood coagulation factor VIII (F8) gene or a composition for the prevention or treatment of hemophilia, which includes: (i) a Cas protein or a nucleotide encoding the Cas protein; and (ii) at least one guide RNA that specifically targets a sequence region of an int22-1/3 homolog or int22-1/2 homolog resulting from conjugation by inversion between homologs 1 (int22-1) and 2 (int22-2) or between homologs 1 (int22-1) and 3 (int22-3) of intron 22 in the blood coagulation factor VIII (F8) gene.

The present invention relates to a method of editing an inversion of the blood coagulation factor VIII (F8) gene or a method of preventing or treating hemophilia, the method including administering, to an individual, (i) a Cas protein or a nucleotide encoding the Cas protein; and (ii) at least one guide RNA that specifically targets a sequence region of an int22-1/3 homolog or int22-1/2 homolog resulting from conjugation by inversion between homologs 1 (int22-1) and 2 (int22-2) or homologs 1 (int22-1) and 3 (int22-3) of intron 22 in the blood coagulation factor VIII (F8) gene.

The present invention also relates to a composition for editing an inversion of the blood coagulation factor VIII (F8) gene, which includes: (i) a Cas protein or a nucleotide encoding the Cas protein; and (ii) at least one guide RNA which specifically targets a sequence region of an int22-1/3 homolog or int22-1/2 homolog resulting from conjugation by inversion between homologs 1 (int22-1) and 2 (int22-2) or between homologs 1 (int22-1) and 3 (int22-3) of intron 22 in the blood coagulation factor VIII (F8) gene.

Another embodiment of the present invention relates to a CRISPR/Cas system for editing an inversion of the blood coagulation factor VIII (FVIII) gene, which uses a guide RNA that targets at least one sequence selected from sequences represented by SEQ ID NOS: 1 to 42 and specifically targets the corresponding sequence, and a Cas protein.

The guide RNA for reversing an inversion according to the present invention targets the sequence region resulting from conjugation by inversion. The "sequence region" may be a range corresponding to an int22-1/2 homolog where int22-1 and int22-2 homologs are conjugated to each other, and an int22-1/3 homolog where int22-1 and int22-3 homologs are conjugated to each other, and particularly, may be a range including sequences having a maximum size of 12 kb, including 5' and 3' directions with respect to the conjugated position.

At least one guide RNA and the Cas protein which constitute the CRISPR/Cas system of the present invention, wherein the at least one guide RNA targets a sequence region resulting from conjugation between int22-1 and int22-2 or int22-3, which are homologs present in intron 22 (int22) in the FVIII gene, may be intracellularly delivered via (a) a vector including nucleic acid sequences expressing at least one guide RNA and the Cas protein, (b) a ribonucleoprotein (RNP) or RNA-guided engineered nuclease (RGEN) consisting of at least one guide RNA and the Cas protein, and (c) at least one guide RNA and mRNA by which the Cas protein is encoded, but the present invention is not limited thereto.

In the present invention, the Cas protein may be a nuclease selected from Cas3, Cas9, Cpf1, Cas6, and C2c2, particularly the Cas protein of CRISPR/Cas type II, and more particularly a *Campylobacter*-derived Cas protein.

In the present invention, the Cas protein may be a nuclease simply isolated from a microorganism, but may be modified to (a) a variant in which some amino acid sequences are substituted, (b) a fusion protein with an affinity tag, recombinase, transposon, or the like, or (c) a variant to which nuclear localization sequences (NLS) are added, but the present invention is not limited thereto.

In the present invention, the guide RNA may consist of (a) crRNA including a guide sequence and tracrRNA, (b) a fusion body of crRNA including a guide sequence and tracrRNA (chimeric guide RNA), or (c) crRNA including a guide sequence, but the present invention is not limited thereto.

The CRISPR/Cas system of the present invention may be included in a composition for editing an inversion of an int22-1/2 or int22-1/3 homolog of the FVIII gene for the treatment of hemophilia A.

The composition for editing an inversion enables normal FVIII expression by genetic correction using the CRISPR/Cas system, and thus may induce a semi-permanent treatment effect for hemophilia A.

A target disease to be treated using the composition of the present invention is severe hemophilia A, which corresponds to symptoms in the case where the activity of FVIII is less than 1% that of normal individuals.

The components of the CRISPR/Cas system included in the composition, i.e., at least one guide RNA targeting a sequence region of an int22-1/3 homolog or int22-1/2 homolog present in intron 22 (int22) in the FVIII gene, and a Cas protein may be intracellularly delivered via (a) a vector including nucleic acid sequences expressing at least one guide RNA and the Cas protein, (b) a ribonucleoprotein (RNP) consisting of at least one guide RNA and the Cas protein, or (c) at least one guide RNA and mRNA by which the Cas protein is encoded, but the present invention is not limited thereto.

In the CRISPR/Cas system of the present invention and the composition using the same, the vector may be one or more vectors, and may be a plasmid vector or a viral vector, and the viral vector may be, particularly, an adenovirus vector, an adeno-associated virus vector, a lentivirus vector, or a retrovirus vector, but the present invention is not limited thereto, and particularly, the viral vector may be an adeno-associated virus vector.

The vectors of the present invention may be delivered to a cell by microvehicles, liposomes, exosomes, nanoparticles, a gene gun, or the like, but the present invention is not limited thereto, and it will be obvious to those of ordinary skill in the art to select a delivery method suitable for the structural characteristics of vectors and the characteristics of cells to which the vectors are to be delivered.

The composition of the present invention may be administered, to a subject in need thereof, in a sufficient or effective amount. The "effective amount" or "sufficient amount" indicates an amount that is used alone or in combination with one or more other therapeutic compositions, protocols, and therapies in a single dose or multiple doses to benefit a subject or provide predicted or desired results for a subject for a certain period of time.

An AAV vector dose for achieving a therapeutic effect may be provided as a vector genome dose/kg (body weight) (vg/kg), but may vary depending on (a) the administration route, (b) expression levels of therapeutic genes required to achieve a therapeutic effect, (c) any host immune response to the AAV vector, and (d) the stability of an expressed protein. When using hemophilia as an example, generally, in order to achieve a therapeutic effect, a blood coagulation factor concentration that is greater than 1% of the factor concentration found in a normal individual is needed in order to change a severe disease phenotype to a moderate phenotype. A severe phenotype is characterized by joint damage and life-threatening bleeding. To convert a moderate disease phenotype into a mild phenotype, a blood coagulation factor concentration greater than 5% of a normal level is required.

The CRISPR/Cas system and composition of the present invention may be incorporated into pharmaceutically acceptable carriers or excipients along with an AAV vector, additional compositions, agents, drugs, and biological agents (proteins), thereby preparing the same as a pharmaceutical composition. In particular, such a pharmaceutical composition is useful for administration and delivery to a patient or subject *in vivo* or *in vitro.*

When prepared as such a pharmaceutical composition, the pharmaceutical composition includes a pharmaceutically acceptable carrier. Pharmaceutically acceptable carriers commonly used in formulation include lactose, dextrose, sucrose, sorbitol, mannitol, starch, acacia gum, calcium phosphate, alginates, gelatin, calcium silicate, microcrystalline cellulose, polyvinylpyrrolidone, cellulose, water, syrup, methyl cellulose, methylhydroxy benzoate, propylhydroxy benzoate, talc, magnesium stearate, mineral oil, saline, phosphate-buffered saline (PBS), or media, but the present invention is not limited thereto. The pharmaceutical composition may further include, in addition to the above-described components, a lubricant, a wetting agent, a sweetener, a flavor enhancer, an emulsifying agent, a suspension agent, a preservative, or the like. Suitable pharmaceutically acceptable carriers and formulations are described in detail in Remington's Pharmaceutical Sciences (19th ed., 1995). A suitable dose of the pharmaceutical composition may be variously prescribed according to factors, such as patient age, body weight, gender, pathological conditions, diet, administration time, administration routes, excretion speed, and reaction sensitivity.

In addition, the pharmaceutical composition may be formulated using a pharmaceutically acceptable carrier and/or excipient by a method that may be easily carried out by one of ordinary skill in the art to which the present invention pertains, to be prepared in a unit dose form or to be contained in a multi-dose container. In this regard, the formulation may be a solution in oil or an aqueous medium, a suspension, a syrup, an emulsion, an extract, powder, granules, tablets, or capsules, and may further include a dispersing agent or a stabilizing agent.

Administration routes for the pharmaceutical composition include oral, transdermal, or local delivery and administration via a systemic, topical, local, or other route, for example, injection, infusion, ingestion, or inhalation. Such delivery and administration routes include parenteral administration such as intravenous administration, intramuscular administration, intraperitoneal administration, intradermal administration, subcutaneous administration, intranasal administration, intracranial administration, transdermal or topical administration, mucosal administration, or intrarectal administration. Examples of administration and delivery routes include intravenous (i.v.) administration and delivery, intraperitoneal (i.p.) administration and delivery, intraarterial administration and delivery, intramuscular administration and delivery, parenteral administration and delivery, subcutaneous administration and delivery, intrapleural administration and delivery, topical administration and delivery, transdermal administration and delivery, intradermal administration and delivery, percutaneous administration and delivery, intracranial administration and delivery, intraspinal administration and delivery, oral (digestive) administration and delivery, transmucosal administration and delivery, respiratory administration and delivery, intranasal administration and delivery, administration and delivery via intubation, intrapulmonary administration and delivery, administration and delivery via intrapulmonary instillation, buccal administration and delivery, sublingual administration and delivery, intravascular administration and delivery, intraarterial administration and delivery, intraluminal administration and delivery, iontophoretic administration and delivery, intraocular administration and delivery, administration and delivery via the eye, intraglandular administration and delivery, intratracheal administration and delivery, and intralymphatic administration and delivery. Delivery and administration doses may be based on existing protocols, and may be determined selectively in human clinical trials or using animal disease models or experimentally. Doses in the initial study may be based on animal studies described herein for mice or dogs, for example.

The subject to be treated with the CRISPR/Cas system or the composition according to the present invention includes animals, typically mammals such as humans, non-human primates (apes, gibbons, gorillas, chimpanzees, orangutans, macaques), pets (dogs and cats), livestock (poultry such as chickens and ducks, horses, cows, goats, sheep, pigs), laboratory animals (mice, rats, rabbits, guinea pigs), and animal disease models, e.g., animal models including mice and other animals with blood-clotting diseases and other diseases known to those of ordinary skill in the art.

Each composition and process used in the present invention has already been described above, and thus descriptions thereof will be omitted herein to avoid excessively repeated descriptions.

Hereinafter, the present invention will be described in further detail with reference to the following examples. It will be obvious to those of ordinary skill in the art that these examples are provided for illustrative purposes only and are not intended to limit the scope of the present invention.

### Examples

### Example 1: Preparation of CRISPR/Cas System

### 1) Selection of Guide RNAs

CRISPR RGEN Tools (http://www.rgenome.net; Park et al., Bioinformatics 31:4014-4016, 2015) was used to select guide RNAs having 5'-NNNNRYAC-3' as a PAM sequence and present in all three regions of the human FVIII gene, i.e., Chr-X:154109091-154118603 (Consortium Human Build 37 (Grch37) standard) corresponding to the int22-1 homolog, Chr-X:154606156-154615709 present apart therefrom by approximately 500 kb and corresponding to the int22-2 homolog, and Chr-X:154684313-154693870 corresponding to the int22-3 homolog. Subsequently, guide RNAs without 1 base or 2 base mismatch except for on-target sites on the human genome were further selected and used for an activity test.

### 2) On-Target/Off-Target Effects of Guide RNAs

Each cloned pU6-CjSgRNA was transfected into HEK293, which is a human cell line, along with pCMV-CjCas9 using Lipofectamine 2000. After about 2 to 3 days, genomic DNA was extracted therefrom and on-target sites were amplified by PCR, and then additional PCR for ligation between adapters specific to sequencing primers for next-generation sequencing and TruSeq HT dual index primers was carried out. Thereafter, reads obtained through paired sequencing were analyzed to confirm insertions or deletions (Indels) at on-target genome locations, through which guide RNAs with activity were selected.

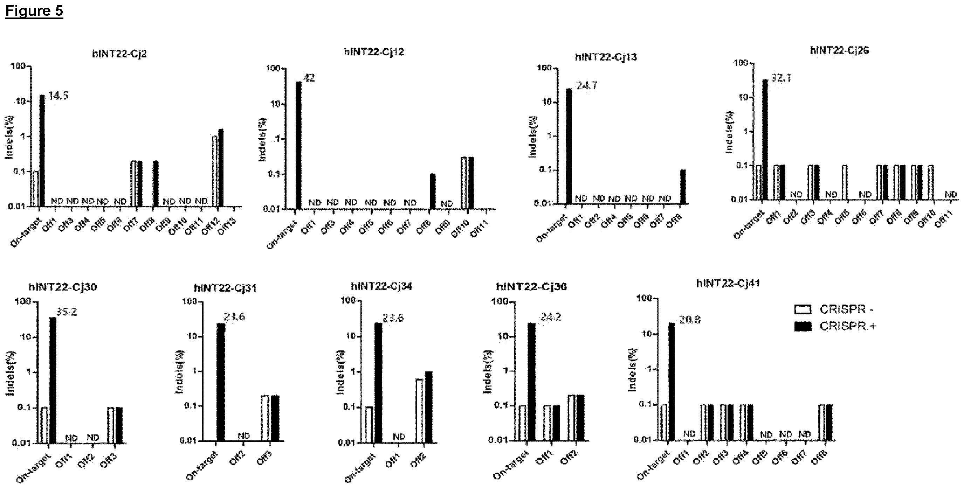

For off-target analysis of the selected guide RNAs, first, off-target lists with 3-base mismatch were selected by an *in-silico* method using Cas-Offinder of CRISPR RGEN Tools, a specific region on the genome corresponding to each off-target was verified through targeted deep sequencing. As a second method, human whole genomic DNA treated with each guide RNA and the CjCas9 protein at 37 °C overnight was subjected to whole genome sequencing, and then potential lists were obtained through Digenome-seq analysis. Thereafter, targeted-deep sequencing was performed on a specific region on the genome of each of off-target candidates to verify whether indels were introduced at off-target positions. In addition, next generation sequencing (NGS) was performed on a specific region on the genome of each of off-target candidates to verify whether indels were introduced at off-target positions. Off-target lists are shown in Tables 3 to 11 below. As illustrated in FIG. 5, it can be confirmed that there was no significant off-targeting in consideration of up to 4 base mismatch on the selected targets.

**[Table 3]**

| **Mismatch (base )** | | **Target sequence-PA**M | **SEQ ID N o.** | **Chr** | **Location** | **Direction** |
|---|---|---|---|---|---|---|
| mismatch (0) | Cj2-ON | GTATACTGTGTTAAGCATTGA-GACAA CAC | 43 | X | | |
| mismatch (4) | Off1 | GTATAaTGTGTaAttCATTGA-CCTAATAC | 44 | chr8 | 99961923 | - |
| | Off2 | GTgTACTtTGcTAAGCtTTGA-TTATACAC | 45 | chr3 | 175280721 | - |
| | Off3 | GTATACTGTtTTAAGgtTTGt-ATCTGTAC | 46 | chr7 | 17171284 | + |
| | Off4 | cTATAaTGTGTTAAGtgTTGA-GAATACAC | 47 | chr7 | 121479898 | - |
| | Off5 | cTATtCTtTGTTAAGCATTtA-CTAAGTAC | 48 | chr5 | 138186574 | + |
| | Off6 | GTgTACTGTGTcAtGCATTGt-CTAAGCAC | 49 | chr5 | 162926700 | - |
| | Off7 | cTATACTGTGccAAGCATTGt-TCAGGTAC | 50 | chr1 | 235243115 | - |
| | Off8 | tTATAaTGTGTTAtGCAgTGA-CTGAACAC | 51 | chr13 | 58076595 | - |
| | Off9 | GTATACTGTGTgcAGCAaaGA-ATTAACAC | 52 | chr2 | 142505968 | + |
| | Off10 | GTATACTGgGTTAAGCccTaA-ACTAGTAC | 53 | chr2 | 225054936 | - |
| | Off11 | caATACTGTtTTAAGCATTGc-TACTACAC | 54 | chr9 | 112040664 | + |
| | Off12 | aTATAaTGTGTTAgGCATTaA-TGAAGCAC | 55 | chrX | 100444393 | + |
| | Off13 | agAcACTGTGTTAAGCATTaA-ACATACAC | 56 | chr11 | 103313729 | - |

**[Table 4]**

| **Mismatch (base)** | | **Target sequence-PAM** | **SEQ ID No.** | **Chr** | **Location** | **Direction** |
|---|---|---|---|---|---|---|
| mismatch (0) | Cj12-ON | GCATCTGGGGACGGCAGAGGG-TATC ACAC | 57 | X | | |
| mismatch (3) | Off1 | GCATaTGGGGcCGGCAGAGtG-GCTCACA C | 58 | chr8 | 41337333 | - |
| | Off2 | GCATCTctGcACGGCAGAGGG-AAACACA C | 59 | chr7 | 45582779 | - |
| | Off3 | GCATCTGGGGtgGGgAGAGGG-TTGGATA C | 60 | chr9 | 92253496 | + |
| mismatch (4) | Off4 | GgAgCTaGGGAgGGCAGAGGG-AGCAGC AC | 61 | chr12 | 2237879 | - |
| | Off5 | GCAgCTGGtGACaaCAGAGGG-GCCGGCA C | 62 | chr3 | 13066365 | + |
| | Off6 | GCtTCTGGGGACGGCccAGaG-GCAGACA C | 63 | chr1 | 15932696 | - |
| | Off7 | GCAgCTGGGGAtGGaAGAGGc-ATGAACA C | 64 | chr1 | 161986421 | + |
| | Off8 | GCATaTGaGGACGGCAGcaGG-TGAGGCA C | 65 | chr13 | 113844761 | + |
| | Off9 | tCATaTGGGGACGGCAGAGaa-AACGGCA C | 66 | chr2 | 222511138 | - |
| | Off10 | GCcTCTGGGGtCaGCAGAaGG-CACGGCA C | 67 | chr2 | 240624171 | - |
| | Off11 | GCAgCTcGGGACGcCAGAGGc-AAATGCA C | 68 | chr15 | 63441723 | + |

**[Table 5]**

| **Mismatch (ba se)** | | **Target sequence-PAM** | **SEQ ID N o.** | **Chr** | **Location** | **Direction** |
|---|---|---|---|---|---|---|
| mismatch (0) | Cj13-ON | TAGGAAGAGAGGCCTGAAACG-ACA TACAC | 69 | X | | |
| mismatch (3) | Off1 | TAGGAAGAtAGGCCTGccACG-TTTCA CAC | 70 | chr10 | 126477396 | + |
| mismatch (4) | Off2 | cAGGAAGAGAGcCCTGgAAgG-TCTT GCAC | 71 | chr3 | 68403798 | + |
| | Off3 | TAGagAGAGAGcCCTtAAACG-CCAG ATAC | 72 | chr3 | 112100249 | - |
| | Off4 | TAaGAgGAGAtGCCTGAcACG-TGGC ACAC | 73 | chr1 | 244962338 | - |
| | Off5 | TgGGgAGAGgGGCCTGAAACc-CAC AACAC | 74 | chr2 | 235938779 | + |
| | Off6 | TgGGcAGAGAGGCCTGAgACc-TGGA ACAC | 75 | chr19 | 32496611 | - |
| | Off7 | TAaGAAGAGAGaCCTGAActG-ACAC ACAC | 76 | chr6 | 139741563 | - |
| | Off8 | TAtGAAtAGAGGCCaGAAACc-AAGA GCAC | 77 | chr20 | 60943338 | + |

**[Table 6]**

| **Mismatch (base)** | | **Target sequence-PAM** | **SEQ ID No.** | **Chr** | **Location** | **Direction** |
|---|---|---|---|---|---|---|
| mismatch (0) | Cj26-ON | TCTTGAGGCCCACCCGCCCCA-ACG AACAC | 78 | X | | |
| mismatch (3) | Off1 | TCaTtAGGCCCAgCCGCCCCA-CGAA GTAC | 79 | chr4 | 13851509 | + |
| mismatch (4) | Off2 | TCTTGAGGaCaACCCtCCCCc-CAGC ACAC | 80 | chr8 | 1709161 | + |
| | Off3 | TCTTaAGGCCCAgCgtCCCCA-CCATG CAC | 81 | chr3 | 52380671 | + |
| | Off4 | TCcTGAGGCCaACCaGCCCCt-ATTCA CAC | 82 | chr3 | 184577591 | - |
| | Off5 | TCcTGAGGaCaAaCCGCCCCA-GGGC GTAC | 83 | chr7 | 73328692 | - |
| | Off6 | TCaTGAGGCCCcaCCGCCCCg-CCCG GCAC | 84 | chr1 | 3590481 | + |
| | Off7 | TCTgGAGGgCCACCCGCaCCc-CCTC ACAC | 85 | chr2 | 10365768 | - |
| | Off8 | aCcTcAGGCCCACCCGtCCCA-GGTC ACAC | 86 | chr22 | 39131563 | + |
| | Off9 | TCTTaAGGCCCAgCtaCCCCA-CCCTA TAC | 87 | chr15 | 73941008 | + |
| | Off10 | cCcTGAGGCCCACaCaCCCCA-CAGG ATAC | 88 | chr6 | 33430699 | - |
| | Off11 | TCTTGAGGCCCcCCtGCtCCt-TGGTA CAC | 89 | chr11 | 113288464 | - |

**[Table 7]**

| **Mismatch (base)** | | **Target sequence-PAM** | **SEQ ID No.** | **Chr** | **Location** | **Direction** |
|---|---|---|---|---|---|---|
| mismatch (0) | Cj30-ON | GCTGAACGTTACCAGCACCCC-GAGAACAC | 90 | X | | |
| mismatch (4) | Off1 | GCTtAAtGTaACCAGCAtCCC-AACTGCAC | 91 | chr12 | 108947583 | + |
| | Off2 | GCaGAACaTTACCAGtACCCt-CACAACAC | 92 | chr5 | 80572029 | + |
| | Off3 | GgTGAAaaTTACCAcCACCCC-CTGCACAC | 93 | chr11 | 114050717 | + |

**[Table 8]**

| **Mismatch (base)** | | **Target sequence-PAM** | **SEQ ID No.** | **Chr** | **Location** | **Direction** |
|---|---|---|---|---|---|---|
| mismatch (0) | Cj31-ON | TTTATAGACGACGGACACCAA-AACCAC AC | 94 | X | | |
| mismatch (4) | Off1 | TTaATAGAtGAaGcACACCAA-CATGGCAC | 95 | chr3 | 135386579 | + |
| | Off2 | TTgATAGACGcCGGAtgCCAA-GCTGGTAC | 96 | chr10 | 76176232 | - |
| | Off3 | TTTATAGACtACctACACCtA-AAAAGCAC | 97 | chrX | 142567739 | - |

**[Table 9]**

| **Mismatch (base)** | | **Target sequence-PAM** | **SEQ ID No.** | **Chr** | **Location** | **Direction** |
|---|---|---|---|---|---|---|
| mismatch (0) | Cj34-ON | CGGAACGCCATCCTGCTTTGG-GGGAA CAC | 98 | X | | |
| mismatch (3) | Off1 | CtGAAaGCCATCCTGCcTTGG-TTATATAC | 99 | chr2 | 153036292 | + |
| mismatch (4) | Off2 | CGGAACtCCAcCtTGCTcTGG-CCCAGCAC | 100 | chr7 | 75597775 | + |

**[Table 10]**

| **Mismatch (base)** | | **Targetsequence-PAM** | **SEQ ID No.** | **Chr** | **Location** | **Direction** |
|---|---|---|---|---|---|---|
| mismatch (0) | Cj36-ON | AATGCATCCTTTGGGCCGAAG-TTGCAC AC | 101 | X | | |
| mismatch (4) | Off1 | AATGCtTCCTTTGGGCtGAgc-ACACACAC | 102 | chr1 | 71100731 | + |
| | Off2 | AATGCATCCTTccGGtCtAAG-AAACACAC | 103 | chr18 | 10367958 | - |

**[Table 11]**

| **Mismatch (base)** | | **Target sequence-PAM** | **SEQ ID No** | **Chr** | **Location** | **Direction** |
|---|---|---|---|---|---|---|
| mismatch (0) | Cj41-ON | GGGTATCTGAGCATAATTCTA-GGAAAC AC | 104 | X | | |
| mismatch (3) | Off1 | GGGTATCTGAtCtTAcTTCTA-CCCAACAC | 105 | chr14 | 68310240 | - |
| mismatch (4) | Off2 | GGGTATtTGAcCATtATTaTA-TGAGATAC | 106 | chr8 | 124870465 | + |
| | Off3 | GttTATCTGgGCATAATgCTA-CTGCACAC | 107 | chr12 | 22066265 | - |
| | Off4 | GGGTAgCTGAtCATAATaCTg-AAAGACAC | 108 | chr7 | 86399626 | + |
| | Off5 | GtGTATCTGAGgATAATcCTg-GAAGATAC | 109 | chr10 | 5232205 | + |
| | Off6 | tGGTATCTGAGCATAAa TtaA-AAGAGTAC | 110 | chr6 | 70660363 | + |
| | Off7 | taGTATCTGAGCATcATTaTA-GAAGATAC | 111 | chr6 | 107643781 | + |
| | Off8 | GGGgAgCTGAtCATAATTCcA-AAAGACAC | 112 | chr11 | 58355632 | - |

### Example 2: Verification of Inversion-Inducing Efficiency by CRISPR/Cas9 System in Normal Cells

Each of the guide RNAs found to have activity was transfected into HEK293 along with a pU6 plasmid and a pCMV-CjCas9 plasmid using Lipofectamine 2000 (manufacturer), and then genomic DNA was extracted. Subsequently, long-distance PCR (LD-PCR) assay was performed using Expand Long Template PCR system (Roche). LC-PCR was performed in a total volume of 25 µl using 600 ng of genomic DNA, 7.5% DMSO, 310 µM dGTP, 190 µM 7-deaza-dGTP, 500 µM of dATP, dCTP, and dTTP each, 0.2 µM inversion-specific primer, 1 Unit Taq Polymerase, and 10X PCR buffer #2. Thereafter, 15 µl of samples were subjected to agarose electrophoresis to identify inversion-specific PCR bands corresponding to about 10.8 kb.

To quantitatively detect inversion efficiency, a standard curve was made through the LD-PCR results of a sample obtained by mixing genomic DNA obtained from induced pluripotent stem cells (iPSCs) of intron-22-inversion patients and genomic DNA of WT HEK293 in a specific ratio, which was then compared with the LD-PCR results by each guide RNA.

### Example 3: Production of Animal Model with Int22-Inverted Genes of FVIII

sgRNAs of SpCas9, exhibiting activity in Chr-X:75243250-75243500 (GRCm38 standard), which is a FVIII int-22 position of mouse genome, and Chr-X:75562900-75563100, which is an intergenic locus region distanced therefrom by about 320 kb, were selected through screening. The sequences of the selected guide RNAs are as follows.

**[Table 12]**

| **Guide RNA** | **Sequence (5'--->3')** | **SEQ ID NO.** |
|---|---|---|
| mF8-int22-Sp1 | GTGGCCTGGTCAAGCTTATC | 113 |
| mF8-int22-Sp2 | TTTGCAGAACAATCCCCTTT | 114 |
| mF8-int22-Sp3 | TTACTAAGGGCTGAACAAGG | 115 |
| mF8-distal-Sp1 | AGCGGGGCTAACACTGCACA | 116 |
| mF8-distal-Sp2 | GCATACGGGTCAGATGCCTG | 117 |
| mF8-distal-Sp3 | CCAAATACGGCAGGGCATAC | 118 |

Target sequences containing the PAMs of hF8-INT22-Cj12 and hF8-INT22-Cj30 to be used for treatment were knocked in on opposite sides by inverted repeats, and at the same time, a single-stranded oligodeoxynucleotide (ssODN) for inducing inversion with respect to a sequence where double strand break occurs by a guide RNA in mouse genome was synthesized. The sequences of ssODNs are as follows.

**[Table 13]**

| ssODN | Sequence | SEQ ID NO . |
|---|---|---|
| ssODN-1 (mF8 -int22) | acaccatctcactgggtgccatggaaccacccctcatccaaacacaccatttggccagtgacttccagatGGCATCTGGG GACGGCAGAGGGTATCACACGGCTGAACGTTACCAGCACCCCGAGAACACacaaggtacaaactaaa ggtcccaagagggaccttactgaaaagttattgaatttaaagaacaatataag | 119 |
| ssODN-2 (mF8 -distal) | ttgttctattaacccaggttcattaacataaaaatagcattgttccccccagtacaacacaagtacctcctGTGTTCTCGGGGT GCTGGTAACGTTCAGCCGTGTGATACCCTCTGCCGTCCCCAGATGCCtgccctgccgtatttggttgtgcca gcacctcttaccagacagtgatctgaggactcagtggacccagat | 120 |

In the ssODN sequences, capital letters denote a human sequence to be knocked in, and underlined sequences denote homolog arm sequences for inversion. SpCas9 mRNA (50 ng/µl), sgRNA (5 ng/µl each), ssODN-1 (20 ng/µl), and ssODN-2 (20 ng/µl) were simultaneously microinjected into mouse fertilized eggs and implanted into surrogate. Subsequently, newborn F0 mice were subjected to genotyping through PCR, TA-sequencing, and whole-genome sequencing to select a mouse model in which 320 kb containing exon1-22 of FVIII was inverted and a desired human sequence was knocked in on opposite sides of the 320 kb interval by an inverted repeat structure. Thereafter, a bleeding test was performed to determine whether symptoms of hemophilia by inversions were exhibited and ELISA measurement was performed on mouse FVIII, to further carry out verification for a hemophilia disease model.

### Example 4: Production of Vector System for Delivering CRISPR/Cas9 System

### 1) RGEN

A pU6-CjSgRNA vector, including a U6 promoter and the sequence of a sgRNA of *Campylobacter jejuni* from which a guide RNA sequence was excluded, was synthesized and produced. Subsequently, a DNA oligo corresponding to each guide RNA was synthesized and cloned into the pU6-CjSgRNA vector using a BsmBI site, thereby producing a plasmid capable of expressing each of the selected guide RNAs in mammalian cells. A CjSgRNA sequence expressed by the produced plasmid is as follows: 5'-NNNNNNNNNNNNNNNNNNNNNNGUUUUAGUCCCUGAAAAGGGACUAAAAUAAAGAGUUUG CGGGACUCUGCGGGGUUACAAUCCCCUAAAACCGCUUUUU-3' (SEQ ID NO. : 121, N is an arbitrary nucleotide), wherein 22 Ns at the 5'-end correspond to a target sequence. A vector including a CMV promoter and a sequence optimized with a human codon of CjCas9 or SpCas9 was synthesized and produced, and each Cas9 includes NLS and HA tags at the C-terminal thereof and further includes NLS also at the N-terminal thereof according to the type of vector.

### 2) AAV

A vector including, between inverted tandem repeats (ITRs) of AAV2, a U6 promoter, an sgRNA sequence, a promoter for mammalian expression (CMV, EFS or LP1), and Cas9 having NLS and HA tags at the C- or N-terminal thereof and optimized with a human codon was synthesized and produced (pAAV-ITR-sgRNA-Cas9). For the production of an AAV, a vector for the pseudotype of an AAV capsid, pAAV-ITR-sgRNA-Cas9, and a pHelper vector were simultaneously transfected into HEK293 cells in a molar concentration ratio of 1:1:1. After 72 hours, the cells were lysed to obtain virus particles, followed by separation and purification by a step-gradient ultracentrifuge using iodixanol (Sigma-Aldrich), and the AAV were quantitatively measured by a titration method using qPCR.

### Example 5: Confirmation of Indels and Reversed State of FVIII int22-1/3 in Animal Model

Mouse embryonic fibroblasts (MEFs) were isolated from FVIII-inverted mice at 2 to 8 weeks of age in which a human target was knocked in and treated with the AAV at a density of 1x10¹¹ vg to 1x10¹² vg through tail vein injection. After about 5-7 days, genomic DNA was extracted from the cells and PCR was performed thereon using PCR primers capable of amplifying the same by reversion of the inversion. Thereafter, the PCR product was cloned into a TA-vector and the reversion of the inversion was confirmed through sequencing. Similarly, the reversion of the inversion was confirmed *in vivo.* The AAV was injected at a density of 1x10¹¹ vg to 1x10¹² vg into the FVIII-inverted mice at 2 to 8 weeks of age in which a human target was knocked in, through tail vein injection. After 4 weeks or 8 weeks, genomic DNA was extracted from each organ, and PCR was performed thereon using PCR primers capable of amplifying the same by reversion of the inversion, and then the reversion of the inversion was confirmed through sequencing of the PCR product. In addition, genomic DNA extracted from each organ was subjected to a targeted deep-sequencing to confirm *in-vivo* indels by CRISPR.

### [Industrial Applicability]

Hemophilia A is a disease that is treated only by replacement therapies because there is no therapeutic agent for fundamental treatment, and even though techniques for gene therapy via therapeutic gene insertion are applied, it is difficult to deliver a therapeutic gene into the human body due to a limitation in packaging in a vector because the size of the FVIII gene is very large. Recently, to address this problem, methods using a minimal domain of FVIII have entered into clinical trials, but only short-term effects are expected, and thus there is a need to develop a fundamental and long-term treatment method. Genetic correction using a CRISPR/Cas9 system is a more fundamental and long-term treatment method, but commonly used Cas9s are difficult to package in an AAV, which is the most clinically proven delivery means, due to a large size thereof, and due to a relatively short PAM, there is a relatively high possibility of potential off-targeting, and thus existing Cas9s are not suitable for use as a component of a gene therapeutic agent.

A CRISPR/Cas system according to the present invention uses a small size of Cas9 and a guide RNA in accordance therewith and consists of a system capable of easily packaging all components of the CRISPR/Cas system in a single AAV, which is impossible in existing Cas9s having a large size. In addition, the CRISPR/Cas system can induce normal gene expression due to the capability to correct inverted genes, and thus is excellent as a technology for efficiently overcoming mutations of genes having a large size, which are difficult to deliver intracellularly, through genetic correction. In particular, the CRISPR/Cas system can induce normal FVIII expression by reversing an inversion of the FVIII gene, and thus is useful for the treatment of hemophilia A.

### Sequence List Free Text

Electronic file attached.

## Claims

1. A composition for editing an inversion of a blood coagulation factor VIII (F8) gene comprising:
(i) a Cas protein or a nucleotide encoding the Cas protein; and
(ii) at least one guide RNA that specifically targets a sequence region of an int22-1/2 homolog or int22-1/3 homolog resulting from conjugation by inversion between homologs 1 (int22-1) and 2 (int22-2) or between homologs 1 (int22-1) and 3 (int22-3) of intron 22 in the blood coagulation factor VIII (F8) gene.

2. The composition according to claim 1, wherein the guide RNA specifically targets a sequence comprising a proto-spacer-adjacent motif (PAM) sequence 5'-NNNNRYAC-3' and a sequence comprising 1 bp or 2 bp mismatch not present on a human genome.

3. The composition according to claim 1, wherein the guide RNA specifically targets a sequence selected from SEQ ID NOS: 1 to 42.

4. A composition for preventing or treating hemophilia, the composition comprising:
(i) a Cas protein or a nucleotide encoding the Cas protein; and
(ii) at least one guide RNA that specifically targets a sequence region of an int22-1/3 homolog or int22-1/2 homolog resulting from conjugation by inversion between homologs 1 (int22-1) and 2 (int22-2) or between homologs 1 (int22-1) and 3 (int22-3) of intron 22 in a blood coagulation factor VIII (F8) gene.

5. The composition according to claim 4, wherein the guide RNA specifically targets a sequence comprising a proto-spacer-adjacent motif (PAM) sequence 5'-NNNNRYAC-3' and a sequence comprising 1 bp or 2 bp mismatch not present on a human genome.

6. The composition according to claim 4, wherein the guide RNA specifically targets a sequence selected from SEQ ID NOS: 1 to 42.

7. A composition for inducing an inversion of a blood coagulation factor VIII (F8) gene, the composition comprising:
(i) a Cas protein or a nucleotide encoding the Cas protein; and
(ii) at least one guide RNA that specifically targets a sequence region of an int22-1/3 homolog or int22-1/2 homolog resulting from conjugation by inversion between homologs 1 (int22-1) and 2 (int22-2) or between homologs 1 (int22-1) and 3 (int22-3) of intron 22 in the blood coagulation factor VIII (F8) gene.

8. The composition according to claim 7, wherein the guide RNA specifically targets a sequence comprising a proto-spacer-adjacent motif (PAM) sequence 5'-NNNNRYAC-3' and a sequence comprising 1 bp or 2 bp mismatch not present on a human genome.

9. The composition according to claim 7, wherein the guide RNA specifically targets a sequence selected from SEQ ID NOS: 1 to 42.

10. A guide RNA that specifically targets one or more sequences selected from sequences represented by SEQ ID NOS: 1 to 42.


<110>	Toolgen Inc.

<120>	Composition for Treating Hemophilia A by CRISPR/Cas System of
	Reverting FVIII Gene Inversion

<130>	2080

<150>	US62/564,708
<151>	2017-09-28

<160>	135

<170>	PatentIn version 3.5

<210>	1
<211>	22
<212>	DNA
<213>	Artificial Sequence

<220>
<223>	gRNA


<400>	1
tctcgttggt gttgtctcaa tg	22


<210>	2
<211>	22
<212>	DNA
<213>	Artificial Sequence

<220>
<223>	gRNA


<400>	2
ggtatactgt gttaagcatt ga	22


<210>	3
<211>	22
<212>	DNA
<213>	Artificial Sequence

<220>
<223>	gRNA


<400>	3
ttaggtagta aaatggcaca ca	22


<210>	4
<211>	22
<212>	DNA
<213>	Artificial Sequence

<220>
<223>	gRNA


<400>	4

tcaggtttta gaataccctg ta                                                22

<210>    5
<211>    22
<212>    DNA
<213>    Artificial Sequence

<220>
<223>    gRNA

<400>    5
cttctcatca tgctacagat tc                                                22

<210>    6
<211>    22
<212>    DNA
<213>    Artificial Sequence

<220>
<223>    gRNA

<400>    6
tctcatcatg ctacagattc tt                                                22

<210>    7
<211>    22
<212>    DNA
<213>    Artificial Sequence

<220>
<223>    gRNA

<400>    7
tcatcatgct acagattctt ta                                                22

<210>    8
<211>    22
<212>    DNA
<213>    Artificial Sequence

<220>
<223>    gRNA

<400>    8
cagggttggc atattagtcc gt                                                22

<210>    9
<211>    22
<212>    DNA
<213>    Artificial Sequence

<220>
<223>    gRNA

<400>    9
aatctgcaac agtcggttgt at                                                22


<210>    10
<211>    22
<212>    DNA
<213>    Artificial Sequence

<220>
<223>    gRNA


<400>    10
tgaaattttc agagtataca tc                                                22


<210>    11
<211>    22
<212>    DNA
<213>    Artificial Sequence

<220>
<223>    gRNA


<400>    11
aaggcatttt gtgttaacgg at                                                22


<210>    12
<211>    22
<212>    DNA
<213>    Artificial Sequence

<220>
<223>    gRNA


<400>    12
ggcatctggg gacggcagag gg                                                22


<210>    13
<211>    22
<212>    DNA
<213>    Artificial Sequence

<220>
<223>    gRNA


<400>    13
ataggaagag aggcctgaaa cg                                                22


<210>    14
<211>    22
<212>    DNA
<213>    Artificial Sequence

<220>

<223>    gRNA

<400>    14
cactaccagc ccccacaccc tc                                                22

<210>    15
<211>    22
<212>    DNA
<213>    Artificial Sequence

<220>
<223>    gRNA

<400>    15
accactacca gcccccacac cc                                                22

<210>    16
<211>    22
<212>    DNA
<213>    Artificial Sequence

<220>
<223>    gRNA

<400>    16
cagtcacttg ccgcacgcct cc                                                22

<210>    17
<211>    22
<212>    DNA
<213>    Artificial Sequence

<220>
<223>    gRNA

<400>    17
gaactttccc tttctactgg at                                                22

<210>    18
<211>    22
<212>    DNA
<213>    Artificial Sequence

<220>
<223>    gRNA

<400>    18
tgtggtgacg gcccctcaca ag                                                22

<210>    19
<211>    22
<212>    DNA
<213>    Artificial Sequence

<220>
<223>    gRNA


<400>    19
catgtggtga cggcccctca ca                                              22


<210>    20
<211>    22
<212>    DNA
<213>    Artificial Sequence

<220>
<223>    gRNA


<400>    20
aggggctggg ccctggtcgc ta                                              22


<210>    21
<211>    22
<212>    DNA
<213>    Artificial Sequence

<220>
<223>    gRNA


<400>    21
gcctagctgg gccaagccga ga                                              22


<210>    22
<211>    22
<212>    DNA
<213>    Artificial Sequence

<220>
<223>    gRNA


<400>    22
cgacaggcta ctgagcaccc tt                                              22


<210>    23
<211>    22
<212>    DNA
<213>    Artificial Sequence

<220>
<223>    gRNA


<400>    23
tggcccctgg cgaggactag ct                                              22


<210>    24
<211>    22

<212>    DNA
<213>    Artificial Sequence

<220>
<223>    gRNA


<400>    24
cccccggcct gaacccccgg cc                                                22


<210>    25
<211>    22
<212>    DNA
<213>    Artificial Sequence

<220>
<223>    gRNA


<400>    25
ggggagtggc cctgggtggg aa                                                22


<210>    26
<211>    22
<212>    DNA
<213>    Artificial Sequence

<220>
<223>    gRNA


<400>    26
gtcttgaggc ccacccgccc ca                                                22


<210>    27
<211>    22
<212>    DNA
<213>    Artificial Sequence

<220>
<223>    gRNA


<400>    27
ggctgaacgt taccagcacc cc                                                22


<210>    28
<211>    21
<212>    DNA
<213>    Artificial Sequence

<220>
<223>    gRNA


<400>    28
tttatagacg acggacacca a                                                 21

<210>	29
<211>	22
<212>	DNA
<213>	Artificial Sequence

<220>
<223>	gRNA


<400>	29
agagagtcac attttataga cg	22


<210>	30
<211>	22
<212>	DNA
<213>	Artificial Sequence

<220>
<223>	gRNA


<400>	30
ctgccttgca gtcacgtgat cg	22


<210>	31
<211>	22
<212>	DNA
<213>	Artificial Sequence

<220>
<223>	gRNA


<400>	31
acggaacgcc atcctgcttt gg	22


<210>	32
<211>	22
<212>	DNA
<213>	Artificial Sequence

<220>
<223>	gRNA


<400>	32
aaagcaggat ggcgttccgt tt	22


<210>	33
<211>	22
<212>	DNA
<213>	Artificial Sequence

<220>
<223>	gRNA


<400>	33
aaatgcatcc tttgggccga ag	22

<210>	34
<211>	22
<212>	DNA
<213>	Artificial Sequence

<220>
<223>	gRNA


<400>	34
ctgatcttcg gggctgagat gt	22


<210>	35
<211>	22
<212>	DNA
<213>	Artificial Sequence

<220>
<223>	gRNA


<400>	35
cccctgcaaa aagacgcaaa ac	22


<210>	36
<211>	22
<212>	DNA
<213>	Artificial Sequence

<220>
<223>	gRNA


<400>	36
cactcaactc acagtgacaa cc	22


<210>	37
<211>	22
<212>	DNA
<213>	Artificial Sequence

<220>
<223>	gRNA


<400>	37
cctctcctgt gcttcccatt ac	22


<210>	38
<211>	22
<212>	DNA
<213>	Artificial Sequence

<220>
<223>	gRNA

<400>    38
tgggtatctg agcataattc ta                                                22


<210>    39
<211>    22
<212>    DNA
<213>    Artificial Sequence

<220>
<223>    gRNA


<400>    39
atgtatcaca tcagtatcaa aa                                                22


<210>    40
<211>    22
<212>    DNA
<213>    Artificial Sequence

<220>
<223>    gRNA


<400>    40
tttaacttga aaacaattcc aa                                                22


<210>    41
<211>    22
<212>    DNA
<213>    Artificial Sequence

<220>
<223>    gRNA


<400>    41
ttcgctgcca ccatttccga cg                                                22


<210>    42
<211>    22
<212>    DNA
<213>    Artificial Sequence

<220>
<223>    gRNA


<400>    42
cggactgtga gttaagaata gt                                                22


<210>    43
<211>    29
<212>    DNA
<213>    Artificial Sequence

<220>
<223>    gRNA

<400>    43
gtatactgtg ttaagcattg agacaacac                                          29


<210>    44
<211>    29
<212>    DNA
<213>    Artificial Sequence

<220>
<223>    gRNA


<400>    44
gtataatgtg taattcattg acctaatac                                          29


<210>    45
<211>    29
<212>    DNA
<213>    Artificial Sequence

<220>
<223>    gRNA


<400>    45
gtgtactttg ctaagctttg attatacac                                          29


<210>    46
<211>    29
<212>    DNA
<213>    Artificial Sequence

<220>
<223>    gRNA


<400>    46
gtatactgtt ttaaggtttg tatctgtac                                          29


<210>    47
<211>    29
<212>    DNA
<213>    Artificial Sequence

<220>
<223>    gRNA


<400>    47
ctataatgtg ttaagtgttg agaatacac                                          29


<210>    48
<211>    29
<212>    DNA
<213>    Artificial Sequence

<220>
<223>    gRNA


<400>    48
ctattctttg ttaagcattt actaagtac                                          29


<210>    49
<211>    29
<212>    DNA
<213>    Artificial Sequence

<220>
<223>    gRNA


<400>    49
gtgtactgtg tcatgcattg tctaagcac                                          29


<210>    50
<211>    29
<212>    DNA
<213>    Artificial Sequence

<220>
<223>    gRNA


<400>    50
ctatactgtg ccaagcattg ttcaggtac                                          29


<210>    51
<211>    29
<212>    DNA
<213>    Artificial Sequence

<220>
<223>    gRNA


<400>    51
ttataatgtg ttatgcagtg actgaacac                                          29


<210>    52
<211>    29
<212>    DNA
<213>    Artificial Sequence

<220>
<223>    gRNA


<400>    52
gtatactgtg tgcagcaaag aattaacac                                          29


<210>    53
<211>    29
<212>    DNA

<213>    Artificial Sequence

<220>
<223>    gRNA


<400>    53
gtatactggg ttaagcccta aactagtac                                          29


<210>    54
<211>    29
<212>    DNA
<213>    Artificial Sequence

<220>
<223>    gRNA


<400>    54
caatactgtt ttaagcattg ctactacac                                          29


<210>    55
<211>    29
<212>    DNA
<213>    Artificial Sequence

<220>
<223>    gRNA


<400>    55
atataatgtg ttaggcatta atgaagcac                                          29


<210>    56
<211>    29
<212>    DNA
<213>    Artificial Sequence

<220>
<223>    gRNA


<400>    56
agacactgtg ttaagcatta aacatacac                                          29


<210>    57
<211>    29
<212>    DNA
<213>    Artificial Sequence

<220>
<223>    gRNA


<400>    57
gcatctgggg acggcagagg gtatcacac                                          29


<210>    58

<211>    29
<212>    DNA
<213>    Artificial Sequence

<220>
<223>    gRNA


<400>    58
gcatatgggg ccggcagagt ggctcacac                                          29


<210>    59
<211>    29
<212>    DNA
<213>    Artificial Sequence

<220>
<223>    gRNA


<400>    59
gcatctctgc acggcagagg gaaacacac                                          29


<210>    60
<211>    29
<212>    DNA
<213>    Artificial Sequence

<220>
<223>    gRNA


<400>    60
gcatctgggg tggggagagg gttggatac                                          29


<210>    61
<211>    29
<212>    DNA
<213>    Artificial Sequence

<220>
<223>    gRNA


<400>    61
ggagctaggg agggcagagg gagcagcac                                          29


<210>    62
<211>    29
<212>    DNA
<213>    Artificial Sequence

<220>
<223>    gRNA


<400>    62
gcagctggtg acaacagagg ggccggcac                                          29

<210>    63
<211>    29
<212>    DNA
<213>    Artificial Sequence

<220>
<223>    gRNA


<400>    63
gcttctgggg acggcccaga ggcagacac                                          29


<210>    64
<211>    29
<212>    DNA
<213>    Artificial Sequence

<220>
<223>    gRNA


<400>    64
gcagctgggg atggaagagg catgaacac                                          29


<210>    65
<211>    29
<212>    DNA
<213>    Artificial Sequence

<220>
<223>    gRNA


<400>    65
gcatatgagg acggcagcag gtgaggcac                                          29


<210>    66
<211>    29
<212>    DNA
<213>    Artificial Sequence

<220>
<223>    gRNA


<400>    66
tcatatgggg acggcagaga aaacggcac                                          29


<210>    67
<211>    29
<212>    DNA
<213>    Artificial Sequence

<220>
<223>    gRNA


<400>    67

gcctctgggg tcagcagaag gcacggcac                                          29

<210>    68
<211>    29
<212>    DNA
<213>    Artificial Sequence

<220>
<223>    gRNA

<400>    68
gcagctcggg acgccagagg caaatgcac                                          29

<210>    69
<211>    29
<212>    DNA
<213>    Artificial Sequence

<220>
<223>    gRNA

<400>    69
taggaagaga ggcctgaaac gacatacac                                          29

<210>    70
<211>    29
<212>    DNA
<213>    Artificial Sequence

<220>
<223>    gRNA

<400>    70
taggaagata ggcctgccac gtttcacac                                          29

<210>    71
<211>    29
<212>    DNA
<213>    Artificial Sequence

<220>
<223>    gRNA

<400>    71
caggaagaga gccctggaag gtcttgcac                                          29

<210>    72
<211>    29
<212>    DNA
<213>    Artificial Sequence

<220>
<223>    gRNA

<400>    72
tagagagaga gcccttaaac gccagatac                                          29


<210>    73
<211>    29
<212>    DNA
<213>    Artificial Sequence

<220>
<223>    gRNA


<400>    73
taagaggaga tgcctgacac gtggcacac                                          29


<210>    74
<211>    29
<212>    DNA
<213>    Artificial Sequence

<220>
<223>    gRNA


<400>    74
tggggagagg ggcctgaaac ccacaacac                                          29


<210>    75
<211>    29
<212>    DNA
<213>    Artificial Sequence

<220>
<223>    gRNA


<400>    75
tgggcagaga ggcctgagac ctggaacac                                          29


<210>    76
<211>    29
<212>    DNA
<213>    Artificial Sequence

<220>
<223>    gRNA


<400>    76
taagaagaga gacctgaact gacacacac                                          29


<210>    77
<211>    29
<212>    DNA
<213>    Artificial Sequence

<220>

<223>    gRNA

<400>    77
tatgaataga ggccagaaac caagagcac                                          29

<210>    78
<211>    29
<212>    DNA
<213>    Artificial Sequence

<220>
<223>    gRNA

<400>    78
tcttgaggcc cacccgcccc aacgaacac                                          29

<210>    79
<211>    29
<212>    DNA
<213>    Artificial Sequence

<220>
<223>    gRNA

<400>    79
tcattaggcc cagccgcccc acgaagtac                                          29

<210>    80
<211>    29
<212>    DNA
<213>    Artificial Sequence

<220>
<223>    gRNA

<400>    80
tcttgaggac aaccctcccc ccagcacac                                          29

<210>    81
<211>    29
<212>    DNA
<213>    Artificial Sequence

<220>
<223>    gRNA

<400>    81
tcttaaggcc cagcgtcccc accatgcac                                          29

<210>    82
<211>    29
<212>    DNA
<213>    Artificial Sequence

<220>
<223>    gRNA


<400>    82
tcctgaggcc aaccagcccc tattcacac                                          29


<210>    83
<211>    29
<212>    DNA
<213>    Artificial Sequence

<220>
<223>    gRNA


<400>    83
tcctgaggac aaaccgcccc agggcgtac                                          29


<210>    84
<211>    29
<212>    DNA
<213>    Artificial Sequence

<220>
<223>    gRNA


<400>    84
tcatgaggcc ccaccgcccc gcccggcac                                          29


<210>    85
<211>    29
<212>    DNA
<213>    Artificial Sequence

<220>
<223>    gRNA


<400>    85
tctggagggc cacccgcacc ccctcacac                                          29


<210>    86
<211>    29
<212>    DNA
<213>    Artificial Sequence

<220>
<223>    gRNA


<400>    86
acctcaggcc cacccgtccc aggtcacac                                          29


<210>    87
<211>    29

<212>    DNA
<213>    Artificial Sequence

<220>
<223>    gRNA


<400>    87
tcttaaggcc cagctacccc accctatac                                         29


<210>    88
<211>    29
<212>    DNA
<213>    Artificial Sequence

<220>
<223>    gRNA


<400>    88
ccctgaggcc cacacacccc acaggatac                                         29


<210>    89
<211>    29
<212>    DNA
<213>    Artificial Sequence

<220>
<223>    gRNA


<400>    89
tcttgaggcc cccctgctcc ttggtacac                                         29


<210>    90
<211>    29
<212>    DNA
<213>    Artificial Sequence

<220>
<223>    gRNA


<400>    90
gctgaacgtt accagcaccc cgagaacac                                         29


<210>    91
<211>    29
<212>    DNA
<213>    Artificial Sequence

<220>
<223>    gRNA


<400>    91
gcttaatgta accagcatcc caactgcac                                         29

<210>    92
<211>    29
<212>    DNA
<213>    Artificial Sequence

<220>
<223>    gRNA


<400>    92
gcagaacatt accagtaccc tcacaacac                                          29


<210>    93
<211>    29
<212>    DNA
<213>    Artificial Sequence

<220>
<223>    gRNA


<400>    93
ggtgaaaatt accaccaccc cctgcacac                                          29


<210>    94
<211>    29
<212>    DNA
<213>    Artificial Sequence

<220>
<223>    gRNA


<400>    94
tttatagacg acggacacca aaaccacac                                          29


<210>    95
<211>    29
<212>    DNA
<213>    Artificial Sequence

<220>
<223>    gRNA


<400>    95
ttaatagatg aagcacacca acatggcac                                          29


<210>    96
<211>    29
<212>    DNA
<213>    Artificial Sequence

<220>
<223>    gRNA


<400>    96
ttgatagacg ccggatgcca agctggtac                                          29

<210>    97
<211>    29
<212>    DNA
<213>    Artificial Sequence

<220>
<223>    gRNA


<400>    97
tttatagact acctacacct aaaaagcac                                          29


<210>    98
<211>    29
<212>    DNA
<213>    Artificial Sequence

<220>
<223>    gRNA


<400>    98
cggaacgcca tcctgctttg ggggaacac                                          29


<210>    99
<211>    29
<212>    DNA
<213>    Artificial Sequence

<220>
<223>    gRNA


<400>    99
ctgaaagcca tcctgccttg gttatatac                                          29


<210>    100
<211>    29
<212>    DNA
<213>    Artificial Sequence

<220>
<223>    gRNA


<400>    100
cggaactcca ccttgctctg gcccagcac                                          29


<210>    101
<211>    29
<212>    DNA
<213>    Artificial Sequence

<220>
<223>    gRNA

<400>    101
aatgcatcct ttgggccgaa gttgcacac                                          29


<210>    102
<211>    29
<212>    DNA
<213>    Artificial Sequence

<220>
<223>    gRNA


<400>    102
aatgcttcct ttgggctgag cacacacac                                          29


<210>    103
<211>    29
<212>    DNA
<213>    Artificial Sequence

<220>
<223>    gRNA


<400>    103
aatgcatcct tccggtctaa gaaacacac                                          29


<210>    104
<211>    29
<212>    DNA
<213>    Artificial Sequence

<220>
<223>    gRNA


<400>    104
gggtatctga gcataattct aggaaacac                                          29


<210>    105
<211>    29
<212>    DNA
<213>    Artificial Sequence

<220>
<223>    gRNA


<400>    105
gggtatctga tcttacttct acccaacac                                          29


<210>    106
<211>    29
<212>    DNA
<213>    Artificial Sequence

<220>
<223>    gRNA

<400>    106
gggtatttga ccattattat atgagatac                                          29


<210>    107
<211>    29
<212>    DNA
<213>    Artificial Sequence

<220>
<223>    gRNA


<400>    107
gtttatctgg gcataatgct actgcacac                                          29


<210>    108
<211>    29
<212>    DNA
<213>    Artificial Sequence

<220>
<223>    gRNA


<400>    108
gggtagctga tcataatact gaaagacac                                          29


<210>    109
<211>    29
<212>    DNA
<213>    Artificial Sequence

<220>
<223>    gRNA


<400>    109
gtgtatctga ggataatcct ggaagatac                                          29


<210>    110
<211>    29
<212>    DNA
<213>    Artificial Sequence

<220>
<223>    gRNA


<400>    110
tggtatctga gcataaatta aaagagtac                                          29


<210>    111
<211>    29
<212>    DNA
<213>    Artificial Sequence

<220>
<223>    gRNA


<400>    111
tagtatctga gcatcattat agaagatac                                          29


<210>    112
<211>    29
<212>    DNA
<213>    Artificial Sequence

<220>
<223>    gRNA


<400>    112
ggggagctga tcataattcc aaaagacac                                          29


<210>    113
<211>    20
<212>    DNA
<213>    Artificial Sequence

<220>
<223>    gRNA


<400>    113
gtggcctggt caagcttatc                                                    20


<210>    114
<211>    20
<212>    DNA
<213>    Artificial Sequence

<220>
<223>    gRNA


<400>    114
tttgcagaac aatccccttt                                                    20


<210>    115
<211>    20
<212>    DNA
<213>    Artificial Sequence

<220>
<223>    gRNA


<400>    115
ttactaaggg ctgaacaagg                                                    20


<210>    116
<211>    20
<212>    DNA

<213>    Artificial Sequence

<220>
<223>    gRNA


<400>    116
agcggggcta acactgcaca                                                    20


<210>    117
<211>    20
<212>    DNA
<213>    Artificial Sequence

<220>
<223>    gRNA


<400>    117
gcatacgggt cagatgcctg                                                    20


<210>    118
<211>    20
<212>    DNA
<213>    Artificial Sequence

<220>
<223>    gRNA


<400>    118
ccaaatacgg cagggcatac                                                    20


<210>    119
<211>    200
<212>    DNA
<213>    Artificial Sequence

<220>
<223>    gRNA


<400>    119
acaccatctc actgggtgcc atggaaccac ccctcatcca aacacaccat ttggccagtg      60

acttccagat ggcatctggg gacggcagag ggtatcacac ggctgaacgt taccagcacc     120

ccgagaacac acaaggtaca aactaaaggt cccaagaggg accttactga aaagttattg     180

aatttaaaga acaatataag                                                 200


<210>    120
<211>    200
<212>    DNA
<213>    Artificial Sequence

<220>
<223>    gRNA

<400>    120
ttgttctatt aacccaggtt cattaacata aaaatagcat tgttccccca gtacaacaca    60

agtacctcct gtgttctcgg ggtgctggta acgttcagcc gtgtgatacc ctctgccgtc    120

cccagatgcc tgccctgccg tatttggttg tgccagcacc tcttaccaga cagtgatctg    180

aggactcagt ggacccagat                                                200


<210>    121
<211>    56
<212>    DNA
<213>    Artificial Sequence

<220>
<223>    gRNA


<400>    121
gagcccgaaa agggacaaaa aaagaggcgg gaccgcgggg acaaccccaa aaccgc        56


<210>    122
<211>    29
<212>    DNA
<213>    Artificial Sequence

<220>
<223>    gRNA


<400>    122
gcatctgggg acggcagagg gtatcacac                                      29


<210>    123
<211>    29
<212>    DNA
<213>    Artificial Sequence

<220>
<223>    gRNA


<400>    123
gcatatgggg ccggcagagt ggctcacac                                      29


<210>    124
<211>    28
<212>    DNA
<213>    Artificial Sequence

<220>
<223>    gRNA


<400>    124
ggaaatgggg acagcagagg tctaacac                                       28

<210>    125
<211>    29
<212>    DNA
<213>    Artificial Sequence

<220>
<223>    gRNA


<400>    125
caaagagggg acggtagagg gttcaatac                                          29


<210>    126
<211>    29
<212>    DNA
<213>    Artificial Sequence

<220>
<223>    gRNA


<400>    126
tggccagggg acagcagagg gagagacac                                          29


<210>    127
<211>    28
<212>    DNA
<213>    Artificial Sequence

<220>
<223>    gRNA


<400>    127
cccaggaggg acgcagagag ctgcacac                                           28


<210>    128
<211>    28
<212>    DNA
<213>    Artificial Sequence

<220>
<223>    gRNA


<400>    128
ctaagagggg acagcagagg ggacacac                                           28


<210>    129
<211>    29
<212>    DNA
<213>    Artificial Sequence

<220>
<223>    gRNA


<400>    129
agacatggag acagcagagg gagagacac                                          29

<210> 130
<211> 29
<212> DNA
<213> Artificial Sequence

<220>
<223> gRNA


<400> 130
agggagaggg acggtagagg gtttagtac 29


<210> 131
<211> 29
<212> DNA
<213> Artificial Sequence

<220>
<223> gRNA


<400> 131
gctgaacgtt accagcaccc cgagaacac 29


<210> 132
<211> 29
<212> DNA
<213> Artificial Sequence

<220>
<223> gRNA


<400> 132
tgccaggtgt cccaccaccc cacgcacac 29


<210> 133
<211> 29
<212> DNA
<213> Artificial Sequence

<220>
<223> gRNA


<400> 133
gcagaacatt accagcaccc cagaaccac 29


<210> 134
<211> 28
<212> DNA
<213> Artificial Sequence

<220>
<223> gRNA

<400>    134
agcaaacgtt accagcaccc atcaacac                                          28

<210>    135
<211>    29
<212>    DNA
<213>    Artificial Sequence

<220>
<223>    gRNA

<400>    135
agaaaacatt accagcaccc cagaagcac                                         29